(19) Europäisches Patentamt European Patent Office Office européen des brevets

(11) **EP 2 012 116 A1**

(12) **EUROPEAN PATENT APPLICATION**
published in accordance with Art. 153(4) EPC

(43) Date of publication:
**07.01.2009 Bulletin 2009/02**

(51) Int Cl.:
***G01N 27/447*** *(2006.01)*    ***C12M 1/00*** *(2006.01)*
***C12Q 1/68*** *(2006.01)*

(21) Application number: **07741491.0**

(22) Date of filing: **12.04.2007**

(86) International application number:
**PCT/JP2007/058055**

(87) International publication number:
**WO 2007/119779 (25.10.2007 Gazette 2007/43)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **14.04.2006 JP 2006112600**

(71) Applicant: **NEC Corporation**
**Minato-ku**
**Tokyo 108-8001 (JP)**

(72) Inventors:
• **ASOGAWA, Minoru**
  **Tokyo 108-8001 (JP)**
• **SUGISAWA, Masatoshi**
  **Tokyo 108-8001 (JP)**
• **OKUI, Shinji**
  **Tokyo 108-8001 (JP)**

(74) Representative: **Merkau, Bernhard et al**
**Glawe. Delfs. Moll**
**Postbox 26 01 62**
**80058 Munich (DE)**

(54) **INDIVIDUAL DISCRIMINATION METHOD AND APPARATUS**

(57)    An individual identification method for identifying an individual by analyzing a DNA sample through electrophoresis, comprises: a first analysis step of analyzing an identifier-attached DNA sample which is given an identifier for an individual; a step of storing a result obtained by analyzing the identifier-attached DNA sample together with a corresponding identifier in a database; a second analysis step of analyzing a new sample which is a DNA sample subjected to individual identification with an accuracy lower than the accuracy when the identifier-attached DNA sample is analyzed, and using the result as a new sample analysis result; and a step of searching the database based on the new sample analysis result.

FIG. 1

EP 2 012 116 A1

**Description**

TECHNICAL FIELD:

The present invention relates to an individual identification method using electrophoresis for DNA (deoxyribonucleic acid), and more particularly to a method and apparatus for accurately identifying an individual using an electrophoretic analyzer having only low reading capabilities.

BACKGROUND ART:

When an individual is identified using DNA for purposes of criminal investigations, i.e., a so-called DNA typing, an analysis is made on a DNA region within a genome which differs from one individual to another. As one method of analyzing DNA, there is electrophoresis which is widely employed. The electrophoresis takes advantages of a flow rate which differs due to the difference in nature of DNA when it is applied with an electric field.

As individual identification using a human's DNA, a method performed by analyzing a region, called "micro-satellite" in which a sequence of approximately four or five bases appears in repetition, has been employed by FBI (Federal Bureau of Inspection), police organization of Japan, and the like. As a method of measuring the number of times of repetitions of micro-satellite regions there is a method of measuring the length of bases of DNA by the electrophoresis. When the electrophoretic measurement of DNA is performed for individual identification, a DNA sequencer which has been much used in DNA determining projects (or genome determining projects) as well is often used as hardware.

The DNA sequencer uses a capillary of approximately 40 cm long filled with gel as a medium for electrophoresis. A solution sample which contains a DNA fragment obtained by amplifying only regions of micro-satellites of DNA and regions adjacent thereto by PCR (Polymerase Chain Reaction) is introduced from one end of the capillary, and the DNA fragment is moved toward the other end of the capillary through electrophoresis which is generated by a force resulting from an electric field. The DNA fragment amplified by PCR is called the "amplicon." In this event, since the moving speed differs depending on the size of the amplicon, i.e., the number of bases in DNA, there is a difference in time from one amplicon to another until it reaches the other end of the capillary. Here, by measuring at which timing the amplicon reaches the other end of the capillary, the size of the DNA related to the amplicon can be estimated, leading to the ability to measure the number of times of repetitions in the micro-satellite region.

This method can be applied not only to the human but also living species which have different DNA regions from one individual to another. In a combined DNA index system (CODIS), which is a DNA profiling system proposed by FBI as a system of identifying human individuals using DNA, and the like, the aforementioned method of analyzing micro-satellites is used, but the number of bases in repetitions of micro-satellites in a gene locus used herein is in units of four bases or five bases.

Other than the analysis on the micro-satellites, there is a method of identifying an individual by fragmenting DNA by a restriction enzyme and analyzing fragments which differ in length. The restriction endonuclease refers to an enzyme which recognizes and cut a particular sequence in DNA. In this method, the electrophoresis can be used for analysis as well.

By the way, an amplicon generated through PCR amplification comprises a repeated sequence portion of targeted micro-satellites, and a portion up to hybridized with a primer of PCR. Therefore, assuming that the number of bases in each repetition is four in a micro-satellite, when the number of repetitions is four in the micro-satellite in a certain amplicon, the micro-satellite portion has 16 (=4×4) bases, and assuming that the number of bases up to hybridized with the primer of PCR is, for example, ten bases, the base length of the amplicon is 26 (=10+16) bases. Likewise, assuming that the number of repetitions of micro-satellites is five, there are 30 bases. In the following, the number of times of repetitions of micro-satellites is represented by an STR (Short Tandem Repeat) count. For example, when the size of a measured amplicon is 30 bases, the STR count can be determined to be five. Since the STR count corresponds to the length of bases of an amplicon, it can be said to be base length information on the amplicon.

In the example described above, the length of an amplicon increases in units of four bases (or five bases) such as 30 bases, 34 bases, and the like, as the STR count increments by one. However, in some gene regions used in the human's DNA profiling, repetitions are not sometimes in increments of four bases (or five bases). For example, in some cases, there is a type which has two extra bases in addition to the normal STR. A type which has two extra bases in addition to five repetitions of STR is labeled "5.2." Assuming that an amplicon has 30 bases when the STR counts is five, "5.2" represents 32 bases. Other than xx.2, xx.1, xx.3 and the like exist. Bases which are fractions with respect to repetitions in this way do not exist in all STR counts, but occur in limited types of, i.e., particular STR counts, as is known in the art.

For example, a locus called FGA has varieties as follows. Table 1 lists examples which show appearing probabilities for locus varieties, showing data on varieties of FGA which was investigated for about 200 African humans in the United States of America. Here, there are 18 types of FGAs, i.e., 18 different STR counts exist for FGA, in which four types are of xx.2 type. In the data shown in Table 1, a total larger than 200 is caused by two types of STR counts derived from a father and a mother, as described below, and a total less than 400 is caused by a failure in analysis. Also, a sum total of appearing probability exceeds 1.0 because the appearance probability is uniformly set to 0.014 when the appearing frequency of STR count is equal to or less than five. The data shown in Table 1 is based on raw data published as "dnaloci.txt" in Bruce Budowle, "Genotype Profiles for Six Population Groups at the 13 CODIS Short Tandem Repeat Core Loci and Other PCRB Based Loci", Forensic Science, Volume 1, Number 2 (July 1999) (Non-Patent Literature 1)

Table 1: Examples of Appearing Frequency of locus varieties

| STR count | Frequency | Probability |
|---|---|---|
| 17.2 | 1 | 0.014 |
| 18 | 3 | 0.014 |
| 18.2 | 3 | 0.014 |
| 19 | 19 | 0.053 |
| 19.2 | 1 | 0.014 |
| 20 | 26 | 0.072 |
| 21 | 45 | 0.125 |
| 22 | 81 | 0.225 |
| 22.2 | 2 | 0.014 |
| 23 | 45 | 0.125 |
| 24 | 67 | 0.186 |
| 25 | 36 | 0.100 |
| 26 | 13 | 0.036 |
| 27 | 8 | 0.022 |
| 28 | 6 | 0.017 |
| 29 | 2 | 0.014 |
| 30 | 1 | 0.014 |
| 30.2 | 1 | 0.014 |
| Total | 360 | 1.073 |

Since there are two sets of human's genome, a father-derived STR count and a mother-derived STR count exist for each locus, and this constitutes information for specifying an individual. Assuming for example that there are ten types of STR counts in a certain locus, 100 (=10×10) types of combinations exist in total. In ten types among them, an STR count in a father-derived locus matches an STR count in a mother-derived locus. Accordingly, even if a DNA analysis is performed for such a human to find the STR count, only one STR count is found. Such a case is called the "homozygosis."

In the remaining 90 types except for the homozygosis, the father-derived STR count differs from the mother-derived STR count. When a DNA analysis is performed for such a human, two STR counts will be found provided that the accuracy is sufficient. Such a case is called the "heterozygosis." For that matter, when a DNA is analyzed, no distinction can be made as to which STR count is derived from the father and which STR count is derived from mother, so that actually, heterozygoses have 45 types, which is one-half of 90 types.

Specifically, when there are ten types of STR counts of loci in each set of each genome, a result which can exist in a DNA analysis has a total of 55 types which is a combination of ten types of homozygoses and 45 types of heterozygoses, and this constitutes information for specifying an individual. In a DNA analysis and reference using micro-satellites, these 55 types are analyzed to pick up which type is pertinent, and an entry which completely matches the analysis result is retrieved from a database.

In the field of DNA-based individual identification, a plurality of loci are analyzed in order to improve the recognition accuracy and retrieve a database. Since the STR count is independently determined for each locus in the human, the recognition accuracy can be increased by analyzing a plurality of loci. In a DNA analysis performed in FBI and the like, 13 loci are used. Details on such DNA analysis is described in detail, for example, in "Forensic DNA Typing, Second Edition: Biology, Technology, and Genetics of STR Markers," John M. Butler, (2005), pp. 85-117, 345-370, and 373-386 (Non-Patent Literature 2).

In this regard, JP-2002-253203-A (Patent Literature 1) discloses that base sequence information of DNA for specifying an individual is digitized and fixed on a bar code or an IC (integrated circuit) card or the like. JP-2003-245098-A (Patent Literature 2) discloses that a PCR product is detected by electrophoresis to find information on the size of a base sequence. JP-2004-073188-A (Patent Literature 3) discloses a method of incorporating a maker into an object to be identified, where the method uses a DNA fragment as the marker. JP-2005-013226-A (Patent Literature 4) discloses a method of identifying a soybean from DNA, where the result of PCR is identified using electrophoresis or the like, and a database is accessed to retrieve satellite DNA upon retrieving a known gene sequence of soybean. JP-2005-160302-A (Patent Literature 5) discloses a gene mapping method using a micro-satellite polymorphic marker. JP-2005-237334-A (Patent Literature 6) discloses a method of rapidly and sensitively measuring a DNA repetition sequence by hybridizing a telomere repetition sequence and a label probe complementary thereto, and detecting the speed of movements of one molecule of its DNA. JP-2005-307216-A (Patent Literature 7) discloses a synthetic DNA ink which can be utilized for authentication of a person. JP-11-118760-A (Patent Literature 8) discloses a method of analyzing an electrophoretic pattern of DNA fragments, which is suitable for creating a database.

WO97/15690 (Patent Literature 9) discloses an invention related to quantification, identification, or determination of a DNA sequence. WO98/35060 (Patent Literature 10) discloses polymerase for analyzing or classifying a polymorphic nuclear acid fragment. WO01/14590 (Patent Literature 11) discloses a method of isolating a defined amount of DNA target substance from another substance within a medium using a silica containing solid support medium, such as silica magnetic particles, having a definable ability to irreversibly couple with a known amount of DNA target substance, and the DNA target substance more than the coupling ability of the particles. WO02/08469 (Patent Literature 12) discloses a method executed by a computer for performing an allele call. WO02/66650 (Patent Literature 13) discloses an analysis on fragments of streptococcus antigen. WO03/06692 (Patent Literature 14) discloses an invention related to an internal calibration standard for electrophoretic analysis. WO02/86794 (Patent Literature 15) discloses a method of analyzing DNA based on mass spectrometry.

In the following, literatures referred to in this description are enumerated:

Patent Literature 1: JP-2002-253203-A.
Patent Literature 2: JP-2003-245098-A.
Patent Literature 3: JP-2004-073188-A.
Patent Literature 4: JP-2005-013226-A.
Patent Literature 5: JP-2005-160302-A.
Patent Literature 6: JP-2005-237334-A.
Patent Literature 7: JP-2005-307216-A.
Patent Literature 8: JP-11-118760-A.
Patent Literature 9: WO97/15690 (JP-2000-500647-A).
Patent Literature 10: WO98/35060 (JP-2001-511018-A).
Patent Literature 11: WO01/14590 (JP-2003-507049-A).
Patent Literature 12: WO02/08469 (JP-2004-516455-A).
Patent Literature 13: WO02/66650 (JP-2004-531235-A).
Patent Literature 14: WO03/06692 (JP-2004-535198-A).
Patent Literature 15: WO02/86794 (JP-2005-509844-A).
Non-Patent Literature 1: Bruce Budowle, "Genotype Profiles for Six Population Groups at the 13 CODIS Short Tandem Repeat Core Loci and Other PCRB Based Loci", Forensic Science, Volume 1, Number 2, (July 1999). (Also available on the Internet from the following URL: <URL.http://www.fbi.gov/hq/lab/fsc/backissu/july1999/budowle.htm>).
Non-Patent Literature 2: "Forensic DNA Typing, Second Edition: Biology, Technology, and Genetics of STR Markers", John M. Butler. (2005). pp. 85-117, 345-370, and 373-386.

DISCLOSURE OF THE INVENTION:

Problems to be Solved by the Invention:

The conventional DNA analysis described above for individual identification need to use large electrophoretic apparatus, giving rise to a problem that a long time is required for electrophoresis to make an analysis time longer. This is because the length of amplicon is measured with accuracy of 1 bp (base pair) in the DNA analysis and the matching with a DNA database. The analysis is made with such a high accuracy of 1 bp in this way because in CODIS proposed by FBI as an individual identification system using human's DNA, or the like, for example, a minimum change width of the DNA size of an amplicon of a locus used herein is approximately 2 bp, so that the matching with a database cannot be accomplished unless the base length is recognized with accuracy of approximately 1 bp.

In order to ensure a measurement accuracy for the length of amplicon, it is not possible to use a capillary shorter than a capillary currently used in an electrophoretic apparatus, or to reduce more the path length of electrophoresis. For this reason, it is not possible to simplify the configuration of the electrophoresis apparatus or the like, or analyze electrophoresis in a short time.

It is an object of the present invention to provide an individual identification method which is capable of ensuring a required accuracy and making an analysis in a short time, even using an electrophoretic apparatus which presents a low reading accuracy.

It is another object of the present invention to provide an individual identification apparatus which is capable of ensuring a required accuracy and making an analysis in a short time, even using an electrophoretic apparatus which presents a low reading accuracy.

Means for Solving the Problem:

Considering an operation of DNA analysis for individual identification, a database has been previously built, and then, DNA of a newly obtained sample is analyzed, and matching is made as to whether or not the analysis result coincides with one stored in the database. Here, processing in a short time, or processing by a simplified apparatus is required by an analysis on DNA of a newly obtained sample, whereas a DNA analysis for data which is previously stored in the database hardly requires the processing by a simplified apparatus or the processing in a short time. Accordingly, the present invention enables a DNA analysis of a newly obtained sample to be performed using an electrophoretic apparatus which is too lowly accurate to be used before in a DNA analysis for individual identification. In the following, the newly obtained sample is called the "new sample."

For reference, a specimen (sample) for registration in a database is clear in identity, i.e., from whom, or when and where it was sampled, and is appended with an identifier for specifying the identity. Accordingly, in the following description, a specimen (sample) for registration in a database is called the "identifier-attached sample." When an identifier-attached sample is stored in a database (i.e., an identifier-attached sample analysis data storage), an DNA analysis may employ a relatively highly accurate electrophoretic apparatus such as one which has been conventionally used, or a relatively lowly accurate electrophoretic apparatus such as one which cannot be conventionally used. As described later, the present invention can accurately accomplish the matching in the database even using a lowly accurate electrophoretic apparatus in both of an analysis on an identifier-attached sample and an analysis on a new sample.

The object of the present invention is achieved by an individual identification method for identifying an individual by analyzing a DNA sample through electrophoresis, which comprises a first analysis step of analyzing an identifier-attached DNA sample which is given an identifier for an individual; a step of storing the result obtained by analyzing the identifier-attached DNA sample together with a corresponding identifier in an identifier-attached sample analysis data storage; a second analysis step of analyzing a new sample which is a DNA sample subjected to individual identification with an accuracy lower than the accuracy when the identifier-attached DNA sample is analyzed, and using the result as a new sample analysis result; and a step of searching the identifier-attached sample analysis data storage based on the new sample analysis result.

In this individual identification method, for example, when the identifier-attached DNA sample and the new sample are analyzed, information related to base lengths of the samples are found through electrophoresis, and particularly, information related to the number of times of repetition of micro-satellites in the sample is captured.

In this individual identification method, typically, the analysis accuracy in the first analysis step is an accuracy with which two DNAs can be identified, where the two DNAs differ in base length by a conceivably minimal amount of change of a base length in the new sample, and the analysis accuracy in the second analysis step is an accuracy with which the two DNA cannot be identified, where the two DNAs differ in base length by the minimum amount of change.

Also, the second analysis step comprises: for example, a step of selecting a plurality of samples in an arbitrary combination from a group of samples each including one type of amplicon, and mixing selected samples to generate a multi-type amplicon sample; a third analysis step of analyzing the multi-type amplicon sample through electrophoresis; a step of storing the result obtain in the third analysis step and base length information of the multi-type amplicon sample in a multi-type amplicon data storage in a paired manner; a fourth analysis step of analyzing the new sample through electrophoresis to obtain new sample electrophoresis result data; and a search step of searching the multi-type amplicon data storage based on the new sample electrophoresis result data, and using the result as the new sample analysis result.

Alternatively, the object of the present invention is achieved by an individual identification method for identifying an individual by analyzing a DNA sample through electrophoresis, which comprises: a first analysis step of analyzing an identifier-attached DNA sample which is given an identifier for an individual to obtain information on a base length of the identifier-attached DNA sample; a step of storing a result obtained by analyzing the identifier-attached DNA sample together with a corresponding identifier in an identifier-attached sample analysis data storage; a second analysis step of analyzing a new sample which is a DNA sample subjected to individual identification, and using the result including information related to a base length of the new sample as a new sample analysis result; and a step of searching the identifier-attached sample analysis data storage based on the new sample analysis result, wherein accuracies in the first analysis step and the second analysis step are accuracies with which two DNAs cannot be identified if the two DNAs differ in base length by a conceivable minimal amount of change of a base length in a DNA sample subjected to individual identification.

The second object of the present invention is achieved by an individual identification apparatus for identifying an individual by analyzing a DNA sample through electrophoresis, which comprises: first analysis means for analyzing an identifier-attached DNA sample which is given an identifier for an individual; an identifier-attached sample analysis data storage for storing the result obtained by analyzing the identifier-attached DNA sample by the first analysis means together with a corresponding identifier; second analysis means having an analysis accuracy lower than the first analysis means, for analyzing a new sample which is a DNA sample subjected to an individual identification, and using the result as a new sample analysis result; and identification means for searching the identifier-attached sample analysis data storage based on the new sample analysis result to obtain an individual identification result.

Alternatively, the second object of the present invention is achieved by an individual identification apparatus for identifying an individual by analyzing a DNA sample through electrophoresis, which comprises: first analysis means for analyzing an identifier-attached DNA sample which is given an identifier for an individual to obtain information on a base length of the identifier-attached DNA sample; an identifier-attached sample analysis data storage for storing the result obtained by analyzing the identifier-attached DNA sample together with a corresponding identifier; second analysis means for analyzing a new sample which is a DNA sample subjected to individual identification, and using the result including information related to a base length of the new sample as a new sample analysis result; and identification means for searching the identifier-attached sample analysis data storage based on the new sample analysis result, wherein accuracies of analysis in the first analysis means and the second analysis means are accuracies with which two DNAs cannot be identified if the two DNAs differ in base length by a conceivable minimal amount of change of a base length in a DNA sample subjected to individual identification.

While the individual identification method and apparatus of the present invention described above analyze DNA samples to perform individual identification, they can further improve the accuracy of the individual identification by combining other biometrics information such as finger print information, palm print information, iris information, face information, and the like.

According to the present invention, since a shorter capillary than currently used capillaries, and a shorter path length of electrophoresis can be used in analyses of a sample subjected to individual identification, i.e., new sample, through electrophoresis, a time required for the analysis is reduced, with the result that DNA based individual identification can be performed in a shorter time.

Also, since such a short capillary and a short path length of electrophoresis are used, the following advantages can be provided.

(1) The apparatus can be simplified in configuration with a reduced size, as compared with the conventional individual identification apparatus, with the result that the DNA based individual identification can be performed at a required location irrespective of indoors or outdoors.
(2) Foreign substances can be readily prevented from introducing from the outside by covering the entire apparatus, and since the apparatus is simple in configuration, it is easy to control external factors which affects electrophoresis, such as temperature, humidity and the like, consequently making it possible to prevent erroneous analyses due to introduction of foreign substances from the outside, and instability of the environment of the analytical instrument.
(3) External factors which affect the apparatus, such as temperature, humidity and the like, are readily controlled by covering the entire apparatus, thus making it possible to improve the maintainability and fault tolerance.

As described above, according to the present invention, DNA-based individual identification can be made in a short time, the DNA-based individual identification can be made at a required location irrespective of indoors or outdoors, and erroneous analyses can be prevented. Consequently, the apparatus of the present invention can be readily combined with another device which performs individual identification using other biometrics information, and the recognition accuracy can be improved by a combination with individual recognition using other biometrics information.

BRIEF DESCRIPTION OF THE DRAWINGS:

FIG. 1 is a diagram showing the configuration of an individual identification apparatus according to a first embodiment of the present invention;

FIG. 2 is a diagram showing the configuration of a low-accuracy electrophoretic analysis unit in the individual identification apparatus shown in FIG. 1;

FIG. 3 is a graph showing the result of a simulation which analyzed a mixture of DNA samples including two types of amplicons;

FIG. 4 is a diagram showing the configuration of a low-accuracy electrophoretic analysis unit in an individual identification apparatus according to a second embodiment of the present invention;

FIG. 5 is a diagram showing the configuration of a low-accuracy electrophoretic analysis unit in an individual identification apparatus according to a third embodiment of the present invention;

FIG. 6 is a diagram showing the configuration of a low-accuracy electrophoretic analysis unit in an individual identification apparatus according to a fourth embodiment of the present invention;

FIG. 7 is a diagram showing the configuration of an individual identification apparatus according to a fifth embodiment of the present invention;

FIG. 8 is a diagram showing the configuration of an individual identification apparatus according to a sixth embodiment of the present invention;

FIG. 9 is a diagram showing the configuration of an individual identification apparatus according to a seventh embodiment of the present invention; and

FIG. 10 is a diagram showing the configuration of an individual identification apparatus according to an eighth embodiment of the present invention.

Description of Reference Numerals:

| | |
|---|---|
| 101 | Uni-type amplicon sample preservation unit |
| 102 | Selected samples |
| 103 | Multi-type amplicon sample |
| 104 | Electrophoretic analysis unit |
| 105 | Multi-type amplicon electrophoresis result data |
| 106 | Multi-type amplicon data storage |
| 107 | New sample |
| 108 | New sample electrophoretic analysis unit |
| 109 | New sample electrophoresis result data |
| 110 | New sample result data analysis unit |
| 111 | New sample analysis result |
| 201 | Interpolation data creation unit |
| 202 | Interpolation data storage |
| 301 | New sample result data analysis unit with parameter estimation function |
| 401 | Uni-type amplicon electrophoresis result data |
| 402 | Uni-type amplicon data storage |
| 403 | Interpolation multi-type amplicon data creation unit |
| 404 | Interpolation multi-type amplicon data storage |
| 501 | Identifier-attached samples |
| 502 | High-accuracy electrophoretic analyzer |
| 503 | Sample analysis result |
| 504 | Identifier-attached sample analysis data storage |
| 505 | Low-accuracy electrophoretic analysis unit |
| 506 | Individual identification unit |
| 507 | Individual identification result |
| 601 | Low-accuracy identifier-attached sample analysis result |
| 602 | Low-accuracy identifier-attached sample analysis data storage |
| 603 | Low-accuracy individual identification unit |
| 604 | Low-accuracy individual identification result |
| 701 | High-accuracy individual identification unit |
| 702 | High-accuracy individual identification result |
| 901 | New sample under acquisition |
| 902 | DNA sample |

| 903 | Individual identification unit based on DNA analysis |
| 904 | Identifier-attached DNA analysis data storage |
| 905 | Individual identification result based on DNA analysis |
| 906 | Finger print sample |
| 907 | Individual identification unit based on finger print analysis |
| 908 | Identifier-attached finger print analysis data storage |
| 909 | Individual identification result based on finger print analysis |
| 910 | Individual identification unit using a plurality of items of information |
| 911 | Individual identification result with a plurality of items of information |
| 1001 | (5, 5) mixed sample |
| 1002 | (5, 5.2) mixed sample |
| 1003 | (5, 6) mixed sample |
| 1004 | (5, 6.2) mixed sample |
| 1005 | (5, 7) mixed sample |
| 1006 | (5, 7.2) mixed sample |
| 1007 | (5, 8) mixed sample |

BEST MODE FOR CARRYING OUT THE INVENTION:

«First Embodiment»

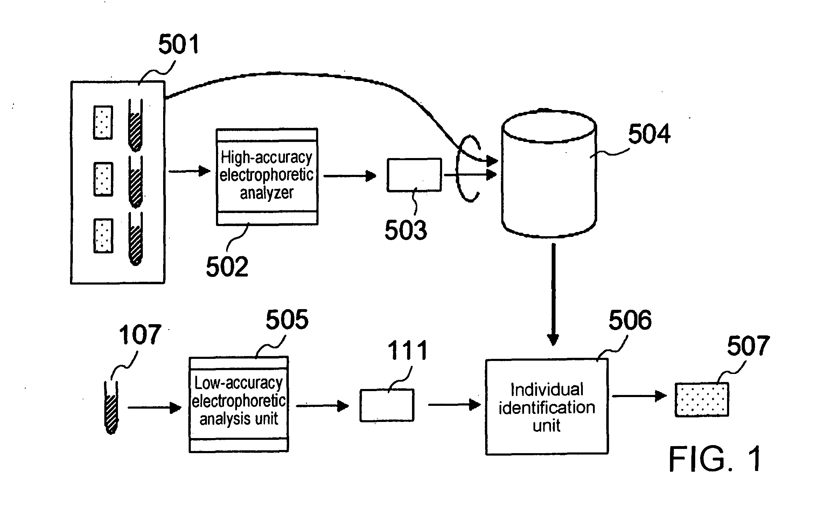

FIG. 1 shows the configuration of an individual identification apparatus according to a first embodiment of the present invention. This individual identification apparatus comprises: high-accuracy electrophoretic analyzer 502 for analyzing identifier-attached samples 501 through electrophoresis; identifier-attached sample analysis data storage 504 for storing sample analysis result 503 supplied from high-accuracy electrophoretic analyzer 502; low-accuracy electrophoretic analysis unit 505 for analyzing new sample 107 through electrophoresis; and individual identification unit 506 for retrieving data within identifier-attached sample analysis data storage 504 based on new sample analysis result 111 supplied from low-accuracy electrophoresis analysis unit 505 to identify an individual for new sample 107, and supplying individual identification result 507. New sample 107 is a sample of DNA for which individual identification is to be performed. The individual identification apparatus of the first embodiment measures STR counts of DNA in new sample 107, and searches a database, i.e., identifier-attached sample analysis data storage 504 based on the measurement result to identify an individual.

Identifier-attached samples 501 are a group of samples to which an identifier of an individual is attached, and high-accuracy electrophoretic analyzer 502 is an apparatus for analyzing each of such identifier-attached samples 501 with a sufficient reading accuracy which has been conventionally used. Sample analysis result 503 is the result of analyzing identifier-attached samples 501 using high-accuracy electrophoretic analyzer 502, and comprises data indicative of a set of a plurality of STR counts within DNA included in identifier-attached samples 501. Identifier-attached sample analysis data storage 504 stores, for each individual of identifier-attached samples 501, a set of a plurality of STR counts, which is sample analysis result 503 analyzed with a sufficient reading accuracy which has been conventionally used, and identifiers of individuals in identifier-attached samples 501 in a paired manner.

While the configuration of low-accuracy electrophoretic analysis unit 505 for analyzing new sample 107 will be described later in detail, low-accuracy electrophoretic analysis unit 505 itself comprises an electrophoretic analyzer. In the first embodiment, this low-accuracy electrophoretic analysis unit 505 is assumed to present a reading accuracy similar to or lower than high-accuracy electrophoretic analyzer 502. New sample analysis result 111 is the result of analyzing new sample 107, and comprises data indicative of a set of a plurality of STR counts. Individual identification unit 506 searches identifier-attached sample analysis data storage 504 for an identifier which has a set of a plurality of STR counts of new sample analysis result 111 that overlaps with a set of STR counts of each entry in identifier-attached sample analysis data storage 504 to create individual identification result 507. Individual identification result 507 may include one individual identifier or a plurality of individual identifiers, or may not at all include any individual identifier.

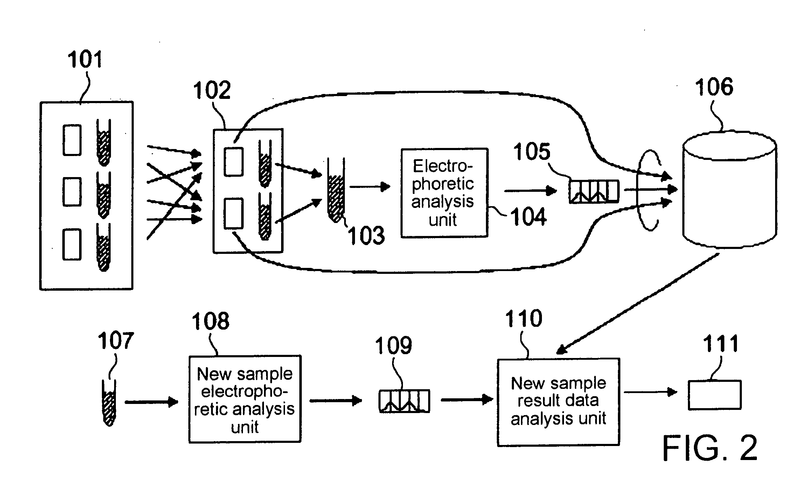

Next, the configuration of low-accuracy electrophoretic analysis unit 505 will be described with reference to FIG. 2.

In the first embodiment, low-accuracy electrophoretic analysis unit 505 comprises: uni-type amplicon sample preservation unit 101; electrophoretic analysis unit 104 for analyzing multi-type amplicon sample 103 produced by mixing DNA samples selected from uni-type amplicon sample preservation unit 101, i.e., selected samples 102, through electrophoresis; multi-type amplicon data storage 106 for storing multi-type amplicon electrophoresis result data 105 supplied from electrophoretic analysis unit 104; new sample electrophoretic analysis unit 108 for analyzing new sample 107 through electrophoresis; and new sample result data analysis unit 110 for searching multi-type amplicon data storage 106 based on new sample electrophoresis result data 109 supplied by new sample electrophoretic analysis unit 108 to deliver a search result as new sample analysis result 111.

Here, uni-type amplicon sample preservation unit 101 preserves a plurality of uni-type amplicon samples, each of which is a DNA sample that includes one type of amplicon, and also holds STR counts in these samples for one amplicon sample to another. Selected samples 102 include (a group of) a plurality of samples selected from uni-type amplicon sample preservation unit 101 in an arbitrary combination. By mixing a plurality of types of selected samples 102 selected in this way, multi-type amplicon sample 103 is produced. Thus, multi-type amplicon sample 103 includes a plurality of types of amplicons which differ in STR counts within a single sample.

In low-accuracy electrophoretic analysis unit 505, multi-type amplicon sample 103 is analyzed by electrophoretic analysis unit 104 to produce multi-type amplicon electrophoresis result data 105 as its result. Multi-type amplicon data storage 106 stores multi-type amplicon electrophoresis result data 105 and an STR count of each amplicon which forms part of multi-type amplicon sample 103 corresponding to that multi-type amplicon electrophoresis data 105 in a paired manner. Also, the result of analyzing new sample 107 by new sample electrophoretic analysis unit 108 is new sample electrophoresis result data 109, and new sample result data analysis unit 110 analyzes an STR count of new sample 107 by retrieving data in multi-type amplicon data storage 106 based on new sample electrophoresis result data 109, and delivers the result of the STR count analysis as new sample analysis result 111.

Next, the operation of this individual identification apparatus will be described.

First, for storing data in a database (i.e., identifier-attached sample analysis data storage 504), each sample of identifier-attached samples 501 is analyzed by high-accuracy electrophoretic analyzer 502 with a sufficient reading accuracy to read information on STR counts in these samples. As a result, since a plurality of STR counts within DNA included in identifier-attached samples 501 are derived as sample analysis result 503, identifier-attached sample analysis data storage 504 stores information which is sample analysis result 503, and identifiers of individuals corresponding to identifier-attached samples 501 in a paired manner.

Next, new samples 107 which are subjected to individual identification are analyzed by low-accuracy electrophoretic analysis unit 505 to obtain new sample analysis result 111 which is a set of a plurality of STR counts. In the following, the processing in low-accuracy electrophoretic analysis unit 505 will be described with reference to FIG. 2.

As described above, since a plurality of DNA samples and their STR counts are preserved in uni-type amplicon sample preservation unit 101, two types or more of the samples are selected from uni-type amplicon sample preservation unit 101 in an arbitrary combination as selected samples 102, and DNA samples of these selected samples 102 are mixed to create a multi-type amplicon sample 103. Then, this multi-type amplicon sample 103 is analyzed through electrophoresis in electrophoretic analysis unit 104 to obtain multi-type amplicon electrophoresis result data 105 as a result. As the result of electrophoresis in electrophoretic analysis unit 103, a peak position of a conical waveform and a shape feature of the conical waveform, or one of them is used. The shape feature of the conical waveform includes one or more of (a) a peak height, (b) a peak width, (c) the area of the conical waveform, and (d) an inflection point of the waveform. Since an approach for analyzing an electrophoresis result is well known to those skilled in the art, and is not directly related to the present invention, a detailed description thereon is omitted.

Once multi-type amplicon electrophoresis result data 105 is obtained, this multi-type amplicon electrophoresis result data 105 and the STR counts in selected samples 102 are stored in multi-type amplicon data storage 106 in a paired manner. Since the STR count is base length information of amplicon as described above, multi-type amplicon data storage 106 stores base length information of multiple types of amplicons. Through such processing, a measurement is made as to which variations are derived from the result of the electrophoretic analysis by a combination of DNA samples of a plurality of types of amplicons, and statistic data is derived. In this regard, while the multi-type amplicon electrophoresis result data is associated with the STR counts, they are simply used as samples for comparison having a plurality of STR counts, and are not directly associated with real individuals.

New samples 107, which are subjected to individual identification, are analyzed by new sample electrophoretic analysis unit 108 using electrophoresis. Here, new sample electrophoretic analysis unit 108 has the same or substantially equivalent analysis performance as or to electrophoresis analysis unit 104 described above. A single electrophoretic analyzer may be shared as electrophoretic analysis unit 104 and new sample electrophoretic analysis unit 108. Since new sample electrophoresis result data 109 is derived as a result by analyzing new samples 107 by new sample electrophoretic analysis unit 108, new sample result data analysis unit 110 retrieves those similar to new sample electrophoresis result data 109 within multi-type amplicon electrophoresis result data 105 stored in multi-type amplicon data storage 106 to analyze STR counts of new samples 107, and delivers the analysis result as new sample analysis result 111.

In the first embodiment, it is assumed, as described above, that the reading accuracy of low-accuracy electrophoretic analysis unit 505 is the same as or lower than high-accuracy electrophoretic analyzer 502. Subsequently, individual identification unit 506 (see FIG. 1) searches for an identifier which has a set of a plurality of STR counts of new sample analysis result 111 that overlaps with a set of STR counts of each entry in identifier-attached sample analysis data storage 504 to produce individual identification result 507. Individual identification result 507 may include one or a plurality of individual identifiers, or may not include any individual identifier, as the case may be.

In the following, a description will be given of how an individual identification result can be obtained with sufficient accuracy even if new samples 107 are analyzed using low-accuracy electrophoretic analysis unit 505 which is not sufficient in reading accuracy.

In the first embodiment, it is assumed that as new sample electrophoretic analysis unit 108 or electrophoretic analysis unit 104 of low-accuracy electrophoretic analysis unit 505, a simple one is used as compared with an electrophoretic analyzer which has been conventionally used in individual identification. In this situation, even if one type of micro-satellite region is to be amplified by PCR, slightly different amplicons can be generated due to incomplete copies of DNA, or even amplicons of the same size can be affected by diffusion during movements through a capillary, so that even if samples from one type of micro-satellite region is electrophoretized, their times of arrival will differ from one to another when they reach the other end of the capillary. As a result, in the electrophoresis result, amplicons distribute over a width with respect to the time of arrival, and its concentration is observed to be a conical waveform. This phenomenon occurs not only when a capillary or the like is used but also when a gel plate is used as a medium of electrophoresis, causing a reduction in accuracy of amplicon size analysis.

When there are two types of amplicons having similar sizes in a DNA micro-satellite region of a human of heterozygosis, two conical waveforms corresponding to different amplicons match with each other, as a result of electrophoresis, and they may be apparently observed as a single conical waveform. However, when the sizes of two types of amplicons largely differ as compared with the widths of the conical waveforms, two conical waveforms appear at different sites, and do not overlap, so that the position and concentration of each conical waveform are correctly analyzed as resulting from the size of each amplicon.

A failure in separating waveforms in close proximity is a problem of the resolution caused by a diffusion or the like during electrophoresis, and in a high-resolution apparatus which prevents the occurrence of such a phenomenon, i.e., an electrophoretic analyzer having a high reading accuracy, conical waveforms have narrower widths in an analysis result, so that the waveforms can be separately observed even if two types of amplicons are substantially the same in size.

When two types of amplicons are substantially the same in concentration, the position of a peak of a conical waveform generated by combining the two types of amplicons is located in the middle of respective peaks of two conical waveforms which are thought to be generated by electrophoresis of the respective amplicons. For example, when two types of amplicons have an STR count of 5, i.e., 30 bases, and an STR count of 5.2, i.e., 32 bases, the conical waveform is observed to have a peak at 31 bases. Assuming that there is a reading error of 2 bp, this sample is recognized as 30 to 32 bases. Therefore, no determination can be made as to whether it has the STR count of 5 or STR count of 5.2.

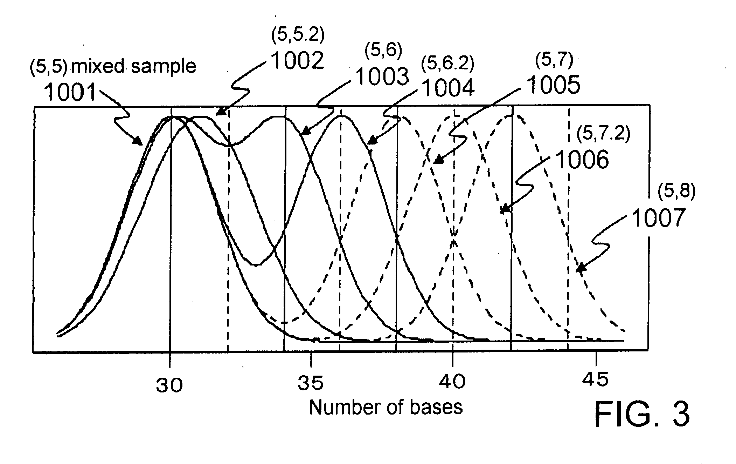

For describing this situation, FIG. 3 shows the result of simulating a result when the shape of a conical waveform is approximated to a Gaussian distribution, and DNA samples including two amplicons which have an STR count of 5 and an STR count of 5 to 8 are mixed, and the resulting mixture is analyzed. In FIG. 3, the x-axis represents the size of DNA. Supposing herein that the repetition unit of STR is 4 bp (base pair), (5, 5) mixed sample 1001 presents the shape of a conical waveform of a mixed sample of (5, 5). Here, an (x, y) mixed sample means that a sample with an STR count of x is mixed with a sample with an STR count of y. When x=y, this represents a homozygosis, whereas when x≠y, this represents a heterozygosis. As can be seen from the simulation of FIG. 3, since the time of arrival varies when amplicons are electrophoretized due to the influence of diffusion or the like, the width of the conical waveform is larger, an influence appears even at a location where the STR count differs by one. In the following, a difference in STR count by one is described by "difference of 1 STR."

In this way, with the degree to which the influence appears at a location at which the STR count differs by one, at a resolution at which the width of the conical waveform is larger, it can be clearly recognized that the DNA samples are samples of heterozygosis for the case of, for example, (5, 7) mixed sample 1005, (5, 7.2) mixed sample 1006, and (5, 8) mixed sample 1007. Accordingly, it seems that at the resolution shown herein, mixed samples which differ by 2 STR or more can be correctly recognized to be a heterozygosis. In this regard, even in (5, 6.2) mixed sample 1004 which differs by 1.2 STRs (i.e., 6 bp), the heterozygosis can be correctly recognized provided that the resolution is 2 bp or finer. It seems that (5, 6) mixed sample 1003 which differs by 1 STR (i.e., 4 bp) can be correctly recognized, if the peak position is relied on, because there is no other similar conical waveform, and the peak position does not shift.

However, at such a resolution, a unimodal conical waveform alone is obtained in (5, 5.2) mixed sample 1002 in which two amplicons differ by 2 bp. Since this conical waveform is similar in shape to a conical waveform of homozygosis ((5, 5) mixed sample 1001), it cannot be specified from the shape of the conical waveform whether it is a heterozygosis or a homozygosis. Since the peak of (5, 5) mixed sample 1001, which is a homozygosis, deviates in position from the peak of (5, 5.2) mixed sample 1002, which is a heterozygosis, by approximately 1 bp, it is possible to recognize the difference between both when the length of DNA can be analyzed with high accuracy, but correct recognition is hard to perform when the accuracy is low with respect to the length of DNA.

In conclusion, in the simulation condition described above, in consideration of both the peculiarity of the shape of the conical waveform and the exactitude of the electrophoresis, a difference by one STR or more can be distinguished, but a homozygosis or a heterozygosis cannot be distinguished with a difference of less than 1 STR. In the first embodiment, it is assumed that new sample electrophoretic analysis unit 108 or electrophoretic analysis unit 104 within low-accuracy electrophoretic analysis unit 505 is low in reading accuracy as compared with the conventional electrophoretic apparatus.

Accordingly, even if identifier-attached sample analysis data storage 504 which stores results based on analyses with high accuracy is directly searched and matched on the basis of data obtained by such low-accuracy electrophoretic analysis unit 505, completely matching data cannot be retrieved in some cases. This is because when an analysis is made by a reader having an accuracy of approximately 2 bp or lower, xx, xx.2 and the like which can appear as STR counts in a human cannot be distinguished

However, assuming that the resolution is approximately 2 bp as in the foregoing example, the STR count can be determined to be 5 or 5.2 provided that a measured base length is approximately 30 bases. In such an event, an entry including a correct STR count can be searched by treating as an STR count of 5 or 5.2 in referencing a database. In this event, however, a problem arises in that assuming that a true STR count is 5, one with STR count of 5.2 is additionally retrieved. Specifically, with new sample electrophoresis result data 109 measured by a device which has a poor resolution, when a search is made in a database produced from high-resolution data, in consideration of those which are possible as STR counts, erroneous entries will be additionally retrieved, although the search can be made including entries of STR counts included in new samples 107. Such a problem of obtaining extra results is hardly problematic in actuality in practical use scenarios by a combination of another approach later described, for example, a method of using multiple types of locus information, or the like.

In the following, a description will be given of why extra results obtained cannot constitute an impediment for individual identification.

Assume herein, for purposes of description, that there are five possible types of STR counts, 4, 5, 5.2, 6, 7 which can occur as a situation, and the respective base lengths are 26, 30, 32, 34, 38 bases.

As an example problem, assume that true STR counts of amplicons of DNA samples (new samples 107) are (5, 5.2). When new sample electrophoresis result data has been obtained at a low resolution (approximately 2 bp), the data cannot be distinguished in some cases which of four types of STR counts {(5, 5), (5, 5.2), (5.2, 5.2), (5.2, 6)} within a database which had been created by performing a read at a high accuracy such as 1 bp, it matches with. In other words, as compared with the case where a device having a resolution of 1 bp is used, the capability to specify the STR count is degraded. However, it can be recognized that the analyzed DNA samples (new samples 107) are not other than the aforementioned four types.

Similarly, assume that true STR counts of amplicons of new samples are (5.2, 5.2). In this event, as a result of matching with the database, it is recognized that it is any of {(5, 5), (5, 5.2), (5.2, 5.2), (5.2, 6), (6, 6)}. While the true STR count (here, 5.2, 5.2)) is included, more STR counts, including the true one, are retrieved. Here, a read error of a peak of a conical waveform, i.e., exactitude is thought to be approximately one base. In other words, even if 34 bases are read, 33, 34, 35 bases are possible as an actual DNA size. The resolution is assumed to be approximately 2 bp. In other words, consider that in the case of a heterozygosis having amplicons which differ by 2 bp, two conical waveforms match so that they are read as a single conical waveform. For example, like the example shown in FIG. 3, in the case of a heterozygosis comprising amplicons, the base lengths of which are 30 bp and 32 bp, a unimodal conical waveform is read with a peak situated about 31 bp. With a heterozygosis, reading errors occur independently of one another with respect to the base length, but when the difference in amplicon size is equal to or less than 4 bp, the amplicons adjoin in a graph of an electrophoresis result, so that a relative reading error of these two amplicons are considered not to be present.

Table 2 shows an example of electrophoresis result of a mixture of two types of amplicons, showing how the mixture of two types of amplicons is analyzed by electrophoresis in the aforementioned situation. "**" in a number at the head of a row indicates that there exist those which have the same pair of sizes of observed DNA. For example, when true STR counts are (4, 5) and (4, 5.2), both cases can be analyzed to be a combination of (25 bp, 31 bp) by the electrophoretic analyzer.

Table 2: Example of Electrophoresis Result of Mixture of Two Types of Amplicons

| | |
|---|---|
| 1: (25bp,25bp) ← (26bp,26bp)=(4,4) | 42: (30bp,33bp) ← (30bp,34bp)=(5,6) |
| 2: (25bp,29bp) ← (26bp,30bp)=(4,5) | 43: (30bp,34bp) ← (30bp,34bp)=(5,6) |
| 3: (25bp,30bp) ← (26bp,30bp)=(4,5) | 44: (30bp,35bp) ← (30bp,34bp)=(5,6) |
| 4: (25bp,31bp) ← (26bp,30bp)=(4,5) | 45: (30bp,37bp) ← (30bp,38bp)=(5,7) |
| **: (25bp,31bp) ← (26bp,32bp)=(4,5.2) | 46: (30bp,38bp) ← (30bp,38bp)=(5,7) |
| 5: (25bp,32bp) ← (26bp,32bp)=(4,5.2) | 47: (30bp,39bp) ← (30bp,38bp)=(5,7) |
| 6: (25bp,33bp) ← (26bp,32bp)=(4,5.2) | 48: (31bp,31bp) ← (30bp,30bp)=(5,5) |
| **: (25bp,33bp) ← (26bp,34bp)=(4,6) | **: (31bp,31bp) ← (30bp,32bp)=(5,5.2) |
| 7: (25bp,34bp) ← (26bp,34bp)=(4,6) | **: (31bp,31bp) ← (32bp,32bp)=(5.2,5.2) |
| 8: (25bp,35bp) ← (26bp,34bp)=(4,6) | 49: (31bp,33bp) ← (30bp,34bp)=(5,6) |
| 9: (25bp,37bp) ← (26bp,38bp)=(4,7) | 50: (31bp,34bp) ← (30bp,34bp)=(5,6) |

(continued)

| | |
|---|---|
| 10: (25bp,38bp) ← (26bp,38bp)=(4,7) | 51: (31bp,35bp) ← (30bp,34bp)=(5,6) |
| 11: (25bp,39bp) ← (26bp,38bp)=(4,7) | 52: (31bp,37bp) ← (30bp,38bp)=(5,7) |
| 12: (26bp,26bp) ← (26bp,26bp)=(4,4) | **: (31bp,37bp) ← (32bp,38bp)=(5.2,7) |
| 13: (26bp,29bp) ← (26bp,30bp)=(4,5) | 53: (31bp,38bp) ← (30bp,38bp)=(5,7) |
| 14: (26bp,30bp) ← (26bp,30bp)=(4,5) | **: (31bp,38bp) ← (32bp,38bp)=(5.2,7) |
| 15: (26bp,31bp) ← (26bp,30bp)=(4,5) | 54: (31bp,39bp) ← (30bp,38bp)=(5,7) |
| **: (26bp,31bp) ← (26bp,32bp)=(4,5.2) | **: (31bp,39bp) ← (32bp,38bp)=(5.2,7) |
| 16: (26bp,32bp) ← (26bp,32bp)=(4,5.2) | 55: (32bp,32bp) ← (30bp,32bp)=(5,5.2) |
| 17: (26bp,33bp) ← (26bp,32bp)=(4,5.2) | **: (32bp,32bp) ← (32bp,32bp)=(5.2,5.2) |
| **: (26bp,33bp) ← (26bp,34bp)=(4,6) | **: (32bp,32bp) ← (32bp,34bp)=(5.2,6) |
| 18: (26bp,34bp) ← (26bp,34bp)=(4,6) | 56: (32bp,37bp) ← (32bp,38bp)=(5.2,7) |
| 19: (26bp,35bp) ← (26bp,34bp)=(4,6) | 57: (32bp,38bp) ← (32bp,38bp)=(5.2,7) |
| 20: (26bp,37bp) ← (26bp,38bp)=(4,7) | 58: (32bp,39bp) ← (32bp,38bp)=(5.2,7) |
| 21: (26bp,38bp) ← (26bp,38bp)=(4,7) | 59: (33bp,33bp) ← (32bp,32bp)=(5.2,5.2) |
| 22: (26bp,39bp) ← (26bp,38bp)=(4,7) | **: (33bp,33bp) ← (32bp,34bp)=(5.2,6) |
| 23: (27bp,27bp) ← (26bp,26bp)=(4,4) | **: (33bp,33bp) ← (34bp,34bp)=(6,6) |
| 24: (27bp,29bp) ← (26bp,30bp)=(4,5) | 60: (33bp,37bp) ← (32bp,38bp)=(5.2,7) |
| 25: (27bp,30bp) ← (26bp,30bp)=(4,5) | **: (33bp,37bp) ← (34bp,38bp)=(6,7) |
| 26: (27bp,31bp) ← (26bp,30bp)=(4,5) | 61: (33bp,38bp) ← (32bp,38bp)=(5.2,7) |
| **: (27bp,31bp) ← (26bp,32bp)=(4,5.2) | **: (33bp,38bp) ← (34bp,38bp)=(6,7) |
| 27: (27bp,32bp) ← (26bp,32bp)=(4,5.2) | 62: (33bp,39bp) ← (32bp,38bp)=(5.2,7) |
| 28: (27bp,33bp) ← (26bp,32bp)=(4,5.2) | **: (33bp,39bp) ← (34bp,38bp)=(6,7) |
| **: (27bp,33bp) ← (26bp,34bp)=(4,6) | 63: (34bp,34bp) ← (32bp,34bp)=(5.2,6) |
| 29: (27bp,34bp) ← (26bp,34bp)=(4,6) | **: (34bp,34bp) ← (34bp,34bp)=(6,6) |
| 30: (27bp,35bp) ← (26bp,34bp)=(4,6) | 64: (34bp,37bp) ← (34bp,38bp)=(6,7) |
| 31: (27bp,37bp) ← (26bp,38bp)=(4,7) | 65: (34bp,38bp) ← (34bp,38bp)=(6,7) |
| 32: (27bp,38bp) ← (26bp,38bp)=(4,7) | 66: (34bp,39bp) ← (34bp,38bp)=(6,7) |
| 33: (27bp,39bp) ← (26bp,38bp)=(4,7) | 67: (35bp,35bp) ← (34bp,34bp)=(6,6) |
| 34: (29bp,29bp) ← (30bp,30bp)=(5,5) | 68: (35bp,37bp) ← (34bp,38bp)=(6,7) |
| 35: (29bp,33bp) ← (30bp,34bp)=(5,6) | 69: (35bp,38bp) ← (34bp,38bp)=(6,7) |
| 36: (29bp,34bp) ← (30bp,34bp)=(5,6) | 70: (35bp,39bp) ← (34bp,38bp)=(6,7) |
| 37: (29bp,35bp) ← (30bp,34bp)=(5,6) | 71: (37bp,37bp) ← (38bp,38bp)=(7,7) |
| 38: (29bp,37bp) ← (30bp,38bp)=(5,7) | 72: (38bp,38bp) ← (38bp,38bp)=(7,7) |
| 39: (29bp,38bp) ← (30bp,38bp)=(5,7) | 73: (39bp,39bp) ← (38bp,38bp)=(7,7) |
| 40: (29bp,39bp) ← (30bp,38bp)=(5,7) | |
| 41: (30bp,30bp) ← (30bp,30bp)=(5,5) | |
| **: (30bp,30bp) ← (30bp,32bp)=(5,5.2) | |

In the format of (pair of observed DNA sizes) <-- (pair of true sizes) = (true STR counts).
** in the number at the head of a row indicates that the same (pair of observed DNA sizes) exists.

Examining DNA data of loci used in a composite DNA index system (CODIS) and the like by FBI, non-integer STR counts, described as xx.1, xx.2, or xx.3 appear a few number of times. In the following description, any of xx.1, xx. 2, and xx.3 is represented by xx.{1, 2, 3}. For example, in the data which shows the variety of locus FGA shown in the aforementioned Table 1, 18 possible STR counts exist, where only four types of them are pertinent to non-integer counts, here to the xx.2 type.

"dnaloci.txt" published in the aforementioned article (Non-Patent Literature 1) by Budowle et al. includes not only data which shows the variety of locus FGA in African Americans, but also data related to similar variety in other loci, other population groups. In the following, a description will be given of how the individual identification apparatus of the first embodiment can correctly identify an individual, using raw data "dnaloci.txt" appended to the article of Budowle et al. Table 3 shows the outline of data used in the following description, showing the relationship between the STR count and appearance frequency on a locus by locus basis. Here, Table 3 shows only those associated with STR counts which are seemingly difficult to analyze at an accuracy of approximately 4 bp.

Table 3: STR Counts and Appearance Frequency on Locus-by-Locus Basis

| Locus | STR counts of xx.{1,2,3} | Appearance Frequency |
|---|---|---|
| CSF1PO | 10.3 | 0.11 |
| D18S51 | 13.2 | 0.17 |
| D18S51 | 14.2 | 0.01 |
| D18S51 | 15.2 | 0.01 |
| D18S51 | 21.2 | 0.02 |
| D21S11 | 24.2 | 0.36 |
| D21S11 | 24.3 | 0.03 |
| D21S11 | 29.2 | 0.12 |
| D21S11 | 30.2 | 2.79 |
| D21S11 | 30.3 | 0.01 |
| D21S11 | 31.2 | 8.72 |
| D21S11 | 32.1 | 0.01 |
| D21S11 | 32.2 | 10.44 |
| D21S11 | 33.2 | 3.42 |
| D21S11 | 34.2 | 0.19 |
| D21S11 | 35.2 | 0.12 |
| D3S1358 | 15.2 | 0.02 |
| D7S820 | 10.1 | 0.01 |
| D7S820 | 11.3 | 0.01 |
| FGA | 17.2 | 0.09 |
| FGA | 18.2 | 0.32 |
| FGA | 19.2 | 0.10 |
| FGA | 20.2 | 0.17 |
| FGA | 21.2 | 0.06 |
| FGA | 22.2 | 0.71 |
| FGA | 22.3 | 0.01 |
| FGA | 23.2 | 0.16 |
| FGA | 24.2 | 0.01 |
| FGA | 24.3 | 0.01 |
| FGA | 30.2 | 0.08 |
| THO1 | 8.3 | 0.11 |
| THO1 | 9.3 | 22.25 |
| Total | | 50.65% ( 13 loci: among 1300 % ) |
| Limited only to STR counts which are seemingly difficult to analyze at an accuracy of approximately 4 bp. | | |

The data used herein include data on six population groups (African American, U.S. Caucasian, Southwestern Hispanic, Bahamian, Jamaican, Trinidadian) in the United States of America. In the following, in order to know average capabilities, assume that as the component ratio of the population groups in the United States of America, the African American occupies 25 %; U.S. Caucasian 45 %; Southwestern Hispanic 20 %; and Bahamian, Jamaican, and Trinidadian the remaining 10 %. Supposing that the proportions of Bahamian, Jamaican, and Trinidadian are 4 %, 4 %, and 2 %, respectively, data is created to proceed with a statistical analysis. Also, representations such as "<xx", ">xx" and the like in the aforementioned raw data, indicate the probabilities of those which have smaller or larger STR counts than xx, but they are omitted because they cause complicated processing and appear a few number of times.

STR counts of xx.{1,2,3} type are included in seven loci (CSF1PO, D18S51, D21 S 11, D3S1358, D7S820, FGA and THO1) in a total of 32 types. Since there are total of 163 types of STR counts, when calculated over all loci, data of xx.{1,2,3} type occupies 19 % as a ratio of type. The appearance ratio of xx.{1,2,3} is 3.85 %.

CODIS itself uses 13 types of loci, and the appearance ratios of xx.{1,2,3} in these loci amount to 50.65 % in total. In this regard, since there are totally 13 loci, the total of frequencies sums up to 1300 %. Focusing attention on frequency data, it can be said that the frequency of xx.{1,2,3} is high in locus D21S11, whereas the frequency of xx.{1,2,3} is very low in the rest of loci, so that STR counts of xx.{1,2,3} type is not encountered so many times. Specifically, it is anticipated that when the STR count is determined to be about 18 using an apparatus which is not capable of distinguishing xx.{1,2,3} from xx, the true STR count is 18 or 18.2, but since the 18.2 appears a number of times as small as 0.014, so that the identification capabilities will hardly change even when 18 and 18.2 are put together into one. In this regard, a correct estimation will be described later.

For correctly estimating recognition capabilities, assume that each STR count of each human appears independently. Consider the probability that STR counts of two humans happen to match in this event. This is a value called "discrimination power" which is an amount indicative of how high a certain analysis approach has recognition capabilities. The recognition capabilities are considered higher as the STR counts of two humans happen to match with a lower probability.

Considering the example of a mixture of data on the six population groups in the United State of America shown in Table 3, the probability that one STR count of FGA is 25 is 0.100, and the probability that it is 24 is 0.186. Accordingly, the probability that FGA of a randomly selected human is (24, 25) is $0.100 \times 0.186 \times 2$. Here, the probability that FGAs of two randomly selected humans happen to be (24, 25) is $(0.100 \times 0.186 \times 2)^2$. The discrimination power when using FGA can be found by the following total sum because this is the probability that both two randomly selected humans for a combination of STR counts related to all FGAs have the same STR count. It should be noted, however, that this is the case of homozygosis, different from the aforementioned example of heterozygosis, where in the case of homozygosis, the appearance probability does not imply a term for doubling, unlike the case of heterozygosis, such as $0.186 \times 0.186$, for example, in the case of (24, 24).

The appearance probabilities are given as follows for the heterozygosis and homozygosis, respectively.

In the case of heterozygosis:

$$\sum_{i,j,i \neq j} \left( \left( Appearance\ probability\ of\ STR\ count\ of\ i \right) \times \left( Appearance\ probability\ of\ STR\ count\ of\ j \right) \times 2 \right)^2$$

In the case of homozygosis:

$$\sum_{i} \left( Appearance\ probability\ of\ STR\ count\ of\ i \right)^4$$

As an accurate estimation, the probability is calculated on the assumption that there are five humans when a combination of STR counts is five humans or less, as described above, but this data supposes that data of six population groups are mixed, so that such a calculation is omitted on the assumption that such accuracy is not required.

In the case of locus FGA, the discrimination power, i.e., the probability that STR counts of two humans happen to match is 0.30391. The discrimination power for other loci used in CODIS is as shown in Table 4. Table 4 shows the discrimination power on a locus-by-locus basis, and the probability that all STR counts of two randomly selected humans match when 13 types of loci are all used.

Table 4: Discrimination Power on Locus-by-Locus Basis, and Probability that All STR Counts of Two Randomly Selected Humans Match when 13 Types of Loci Are All Used

| Locus | Discrimination Power | In -$\log_{10}$ Notation | Number of Types |
|---|---|---|---|
| D13S317 | 0.080887 | (1.092124) | 9 |
| CSF1PO | 0.104326 | (0.981606) | 11 |
| D16S539 | 0.075676 | (1.121040) | 8 |
| D18S51 | 0.025047 | (1.601245) | 19 |
| D21S11 | 0.037495 | (1.426024) | 23 |
| D3S1358 | 0.085779 | (1.066618) | 9 |
| D5S818 | 0.126088 | (0.899327) | 10 |
| D7S820 | 0.070993 | (1.148787) | 11 |
| D8S1179 | 0.066310 | (1.178421) | 11 |
| FGA | 0.030391 | (1.517258) | 26 |
| THO1 | 0.078208 | (1.106751) | 8 |
| TPOX | 0.143799 | (0.842243) | 8 |
| vWA | 0.061754 | (1.209334) | 10 |
| Total | $6.444986 \times 10^{-16}$ | (15.190778) | 163 |

A parenthesized number on the third column from the left of Table 4 indicates the "discrimination power (i.e., the probability that they happen to match)" in -$\log_{10}$ notation. Thus, when the number in parenthesis is 1.0, this means that the STR counts happen to match one in every ten humans. The number on the last column indicates the number of types of STR counts on the locus-by-locus basis. The more the types of STR counts are, the probability of accidental matching is lower. However, even if the number of types of STR counts is the same, there is a bias in the distribution of STR counts, so that the "probability of accidental matching" does not become the same.

When all of 163 types of STR counts are used in the 13 loci described above, the probability that two randomly selected humans happen to match is the product of the discrimination power of the respective loci, calculated as $6.444986 \times 10^{-16}$ ($=10^{-15.190778}$), so that the matching occurs one in every $1.551594 \times 10^{+15}$ ($=1/6.444986 \times 10^{-16}$) humans.

In the first embodiment, the result of an analysis on DNA samples, i.e., new samples 107 is derived as new sample analysis result 1111 using new sample electrophoretic analysis unit 108 or electrophoretic analysis unit 104 within low-accuracy electrophoretic analysis unit 505, and the database is searched on the basis of this analysis result, thereby making it possible to retrieve entries of STR counts included in DNA samples. In this event, as described above, erroneous entries are also retrieved in surplus. As such, consider the "probability that two randomly selected humans happen to match" in a situation in which erroneous entries are also retrieved in surplus.

Assume that due to a low reading accuracy caused by the use of low-accuracy electrophoretic analysis unit 505, xx, xx.2, and xx+1 cannot be distinguished from one another. In other words, consider a situation in which they are "recognized as one type of STR count." Table 5 shows the discrimination power in such a situation in comparison with the discrimination power when the analysis accuracy is approximately 1 bp in electrophoresis, showing the discrimination power for each locus, and the probability that all STR counts of two randomly selected humans match when 13 types of loci are all used, when the low-accuracy electrophoretic analyzer is used.

Table 5: Discrimination Power for Each Locus, and Probability that All STR Counts of Two Randomly Selected Humans Match when 13 Types of Loci Are All Used, when Low-Accuracy Electrophoretic Analyzer Is Used

| Locus | High-Accuracy Electrophoretic Analyzer | | Low-Accuracy Electrophoretic Analyzer | | Difference |
|---|---|---|---|---|---|
| | Discrimination Power | In -$\log_{10}$ Notation | Discrimination Power | In -$\log_{10}$ Notation | |
| D13S317 | 0.080887 | (1.092124) | 0.080887 | (1.092124) | 0 |
| CSF1PO | 0.104326 | (0.981606) | 0.105320 | (0.977490) | 0.004116 |

(continued)

| Locus | High-Accuracy Electrophoretic Analyzer | | Low-Accuracy Electrophoretic Analyzer | | Difference |
| --- | --- | --- | --- | --- | --- |
| | Discrimination Power | In -$\log_{10}$ Notation | Discrimination Power | In -$\log_{10}$ Notation | |
| D16S539 | 0.075676 | (1.121040) | 0.075676 | (1.121040) | 0 |
| D18S51 | 0.025047 | (1.601245) | 0.025463 | (1.594093) | 0.007152 |
| D21S11 | 0.037495 | (1.426024) | 0.059666 | (1.224274) | 0.20175 |
| D3S1358 | 0.085779 | (1.066618) | 0.085946 | (1.065774) | 0.000844 |
| D5S818 | 0.126088 | (0.899327) | 0.126088 | (0.899327) | 0 |
| D7S820 | 0.070993 | (1.148787) | 0.071117 | (1.148027) | 0.00076 |
| D8S1179 | 0.066310 | (1.178421) | 0.066310 | (1.178421) | 0 |
| FGA | 0.030391 | (1.517258) | 0.034277 | (1.465002) | 0.052256 |
| THO1 | 0.078208 | (1.106751) | 0.129458 | (0.887872) | 0.218879 |
| TPOX | 0.143799 | (0.842243) | 0.143799 | (0.842243) | 0 |
| vWA | 0.061754 | (1.209334) | 0.061754 | (1.209334) | 0 |
| Total | | (15.190778) | | (14.75020) | 0.485758 |

The leftmost column in Table 5 indicates a locus name, and the second and third columns from the left indicate the discrimination power when using a high-accuracy electrophoretic analyzer which provides an analysis accuracy of approximately 1 bp, and its representation in -$\log_{10}$ notation. In this regard, values in the second and third columns from the left are the same as those shown in Table 4. The fourth column from the left in Table 5 described "Low-Accuracy Electrophoretic Analyzer" indicates the discrimination power by use of low-accuracy electrophoretic analysis unit 505 which provides a resolution of 4 bp, as described above, and the fifth column indicates the discrimination power in the fourth column in -$\log_{10}$ notation. The rightmost column in Table 5 shows the difference between the third column and the fifth column. Considering a value in the rightmost column represented by c and its 10's power, *i.e.*, $10^c$, the use of low-accuracy electrophoretic analysis unit 505 results in a reduction in discrimination power by $10^c$.

In loci D12S317, D16S539, D5S818, D8S1179, TPOX and vWA, no xx.{1,2,3} type exists in the STR counts, so that even if the accuracy is reduced in electrophoresis as mentioned above, different STR counts can be correctly identified, so that no reduction in discrimination power occurs. This is indicated by the value equal to zero in the rightmost column in Table 5. On the other hand, in locus D21S11 and THO1, the values shown in the rightmost column is approximately 0.2 as a difference in -$\log_{10}$ notation, from which it can be seen that the discrimination power degrades by a factor of 0.63 (=$10^{-0.2}$).

As shown in the lowermost row of Table 5, assuming that all STR counts of 13 loci shown herein are used, and low-accuracy electrophoretic analysis unit 505 such as one described above is used, the probability that two randomly selected humans happen to match is $1.972332 \times 10^{-15}$ (=$10^{-14.705020}$), meaning that the matching is found with a probability of one in every $5.07014 \times 10^{+14}$ humans. The 13 loci used herein are the same as the 13 loci used in CODIS.

On the other hand, the probability that two randomly selected humans happen to match is $6.444986 \times 10^{-16}$ (=$10^{-15.190778}$) when using an electrophoretic analyzer which provides an analysis accuracy of 1 bp, as has been conventionally used, and using all STR counts of the 13 loci, meaning that the matching is found with a probability of one in every $1.551594 \times 10^{+15}$ (=$1/6.444986 \times 10^{-16}$) humans. Thus, it can be seen that with the use of low-accuracy electrophoretic analysis unit 505, the discrimination power exacerbates from $1/(1.551594 \times 10^{+15})$ to $1/(5.07014 \times 10^{+14})$ in the 13 loci used in CODIS and the like. In other words, the discrimination power exacerbates by a factor of 0.3267699.

Considering the fact that objects can be narrowed down to approximately one tenth each time the STR count is used as a search condition on average, when individual identification is performed using 13 loci of CODIS, the difference in recognition capabilities (0.32677699 times) when an electrophoretic analyzer with a resolution of 1 bp is used and when an electrophoretic analyzer with a resolution of 4 bp is used, can be regarded as similar to that when "information on one certain locus was not used," or a degradation in recognition capabilities equal to or lower than that.

The discrimination power can be used to calculate an indicia of "how often an STR count of a sample at hand matches with a certain entry in a database." This value is a value used in courts and the like in order to prove a probative force and the like of an appraisement. The discrimination power is the "probability that both two randomly selected humans have the same individual gene type," whereas this indicia indicates the probability that "an STR count of a sample at hand matches with an entry in a database, but STR counts of samples of other n humans do not match with the database." Assuming herein that p represents the "probability that both two randomly selected humans have the same individual gene type," the probability that "they do not match with the database" is represented by (1-p). Since there are n humans, the probability that all n humans do not match with the database" is represented by $(1-p)^n$. Calculating with a significance level of 1 % or less, such that such a thing itself will hardly occur, $(1-p)^n \geq 1-0.01$ is given.

Applying this situation to the population of the United States of America, n is 300,000,000, and $p \leq 3.33 \times 10^{-11}$ is given, paying attention that $(1-p)^n$ can be approximated to 1-np.

It is necessary to compare $6.444986 \times 10^{-16}$ ($=10^{-15.190778}$) which is the probability that this value happens to match with STR counts of two randomly selected humans when reading at a resolution of 1 bp with $1.972332 \times 10^{-15}$ ($=10^{-14.705020}$) which is the probability that the value happens to match with STR counts of two randomly selected humans when reading at a resolution of 4 bp.

Paying attention to:

$$(1-p)^n \geq 1 - \text{Significance Level,}$$
$$1-np \geq 1 - \text{Significance Level, and}$$
$$\text{Significance Level} \geq np,$$

the significance level when reading at a resolution of 1 bp, and the significance level when reading at a resolution of 4 bp, as shown in the aforementioned condition, are $1.933496 \times 10^{-7}$ ($=6.444986 \times 10^{-16} \times 3 \times 10^8$), and $5.916996 \times 10^{-7}$ ($=1.972332 \times 10^{-15} \times 3 \times 10^8$), respectively. The significance level at the lower resolution is approximately three times higher as compared with the significance level at 1 bp.

Summarizing the foregoing, when an analysis is made at a conventional high resolution, "an STR count of a sample at hand matches an entry in a database, but STR counts of samples of n other humans do not match with the database" can be asserted with a probability of 99.99998 % ($=1.0-1.933496 \times 10^{-7}$). On the other hand, in a situation where xx, xx.2, and xx+1 cannot be distinguished from one another due to a low reading accuracy of low-accuracy electrophoretic analysis unit 505, a difference lies in that "an STR count of a sample at hand matches an entry in a database, but STR counts of samples of n other humans do not match with the database" can be asserted with a probability of 99.99994 % ($=1.0-5.916996 \times 10^{-7}$). In other words, it can be seen that no problem will practically arise because the "probability that two randomly selected humans have the same individual gene type." slightly varies at the fifth decimal place.

«Second Embodiment»

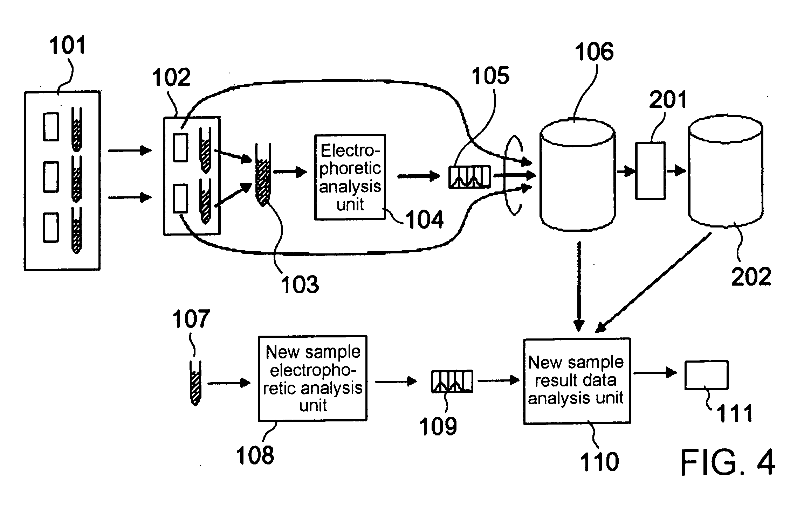

Next, a description will be given of an individual identification apparatus according to a second embodiment of the present invention. While this individual identification apparatus is similar to the individual identification apparatus of the first embodiment shown in FIG. 1, they differ in the configuration of low-accuracy electrophoretic analysis unit 505. FIG. 4 shows the configuration of low-accuracy electrophoretic analysis unit 505 in the individual identification apparatus of the second embodiment.

In the first embodiment described above, multi-type amplicon samples 103 are provided in all combinations in the creation of data which should be stored in multi-type amplicon data storage 106, whereas in the second embodiment, DNA samples (selected samples 102) of STR counts are prepared in proper combinations and they are mixed to produce multi-type amplicon samples 103, instead of preparing multi-type amplicon samples 103 in all combination. Then, multi-type amplicon samples 103 are analyzed by electrophoretic analysis unit 104, and multi-type amplicon electrophoresis result data 105 resulting from the analysis is preserved in multi-type amplicon data storage 106. In this event, while combinations of STR counts with which multi-type amplicon samples 103 are not actually produced exist within possible combinations of amplicons, data is generated with respect to such combinations of STR counts through interpolation or the like using a simulation method or the like from measured data in multi-type amplicon data storage 106. Thus, in the second embodiment, low-accuracy electrophoretic analysis unit 505 comprises interpolation data creation unit 201 for generating data through interpolation from data measured and stored in multi-type amplicon data storage 106, and interpolation data storage 202 for interpolating data generated by interpolation data generation unit 201. New sample result data analysis unit 110 compares and analyzes new sample electrophoresis result data 109, which is the result of analyzing new samples 107 through electrophoresis, and data stored in multi-type amplicon data storage 106 and data stored in interpolation data storage 202 to estimate STR counts of new samples 107, and delivers the results as new sample analysis results 111.

«Third Embodiment»

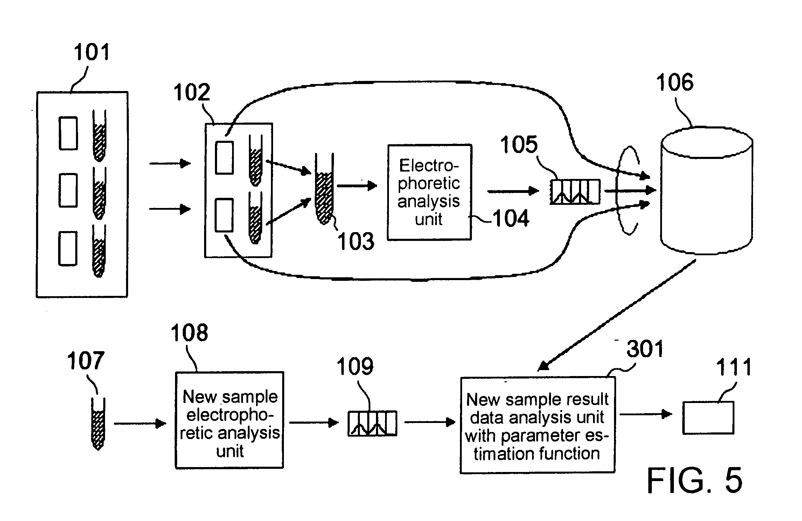

Next, a description will be given of an individual identification apparatus according to a third embodiment of the present invention. While this individual identification apparatus is similar to the individual identification apparatus of the first embodiment shown in FIG. 1, they differ in the configuration of low-accuracy electrophoretic analysis unit 505. FIG. 5 shows the configuration of low-accuracy electrophoretic analysis unit 505 in the individual identification apparatus of the third embodiment.

In the first embodiment described above, multi-type amplicon samples 103 are provided in all combinations in the creation of data which should be stored in multi-type amplicon data storage 106, whereas in the third embodiment, DNA samples (selected samples 102) of STR counts are prepared in proper combinations and they are mixed to produce multi-type amplicon samples 103, instead of preparing multi-type amplicon samples 103 in all combination. Then, multi-type amplicon samples 103 are analyzed by electrophoretic analysis unit 104, and multi-type amplicon electrophoresis result data 105 resulting from the analysis is preserved in multi-type amplicon data storage 106. In this event, combinations of STR counts with which multi-type amplicon samples 103 are not actually produced exist within possible combinations of amplicons, the third embodiment employs new sample result data analysis unit 301 with parameter estimation function, which has a parameter estimation function, as the new sample result data analysis unit.

New sample result analysis unit 301 with parameter estimation function retrieves data in multi-type amplicon data storage 106 based on new sample electrophoretic result data 109 which is the result of analyzing new samples 107 by new sample electrophoretic analysis unit 108, and uses data previously stored in multi-type amplicon data storage 106, when analyzing new sample electrophoresis result data 109, to parameterize the manner of change in new sample electrophoresis result data 109 based on a change in STR counts, for use in analysis. New sample result analysis unit 301 with parameter estimation function analyzes STR counts of new sample electrophoresis result data 109 to deliver new sample analysis result 111.

«Fourth Embodiment»

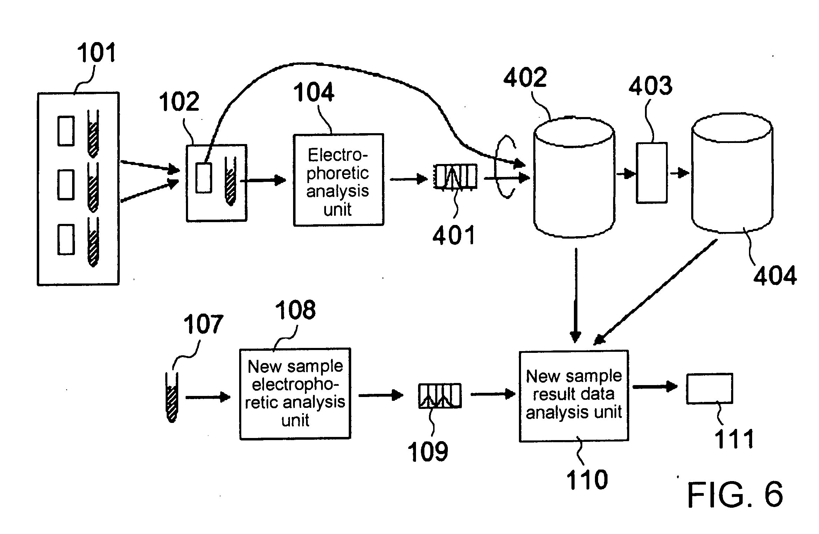

Next, a description will be given of an individual identification apparatus according to a fourth embodiment of the present invention. While this individual identification apparatus is similar to the individual identification apparatus of the first embodiment shown in FIG. 1, they differ in the configuration of low-accuracy electrophoretic analysis unit 505. FIG. 6 shows the configuration of low-accuracy electrophoretic analysis unit 505 in the individual identification apparatus of the fourth embodiment.

The first embodiment generates multi-type amplicon samples 103 which are analyzed through electrophoresis, and stores the result of the analysis in multi-type amplicon data storage 106, whereas the fourth embodiment performs an electrophoretic analysis on uni-type amplicon samples as they are, without generating multi-type amplicon samples, derives and stores analysis results of samples including a plurality of amplicons from the electrophoretic analysis through interpolation, and analyses new sample electrophoresis result data 109 based on the stored result, thereby producing an analysis result for new samples 107 as new sample analysis result 111.

Specifically, in the fourth embodiment, low-accuracy electrophoretic analysis unit 505 comprises: uni-type amplicon sample preservation unit 101; electrophoretic analysis unit 104 for analyzing DNA samples (selected samples 102) selected from uni-type amplicon sample preservation unit 101 through electrophoresis; uni-type amplicon data storage 402 for storing uni-type amplicon electrophoresis result data 401 supplied from electrophoretic analysis unit 104; interpolation multi-type amplicon data creation unit 403 for creating interpolation multi-type amplicon data based on data stored in uni-type amplicon data storage 402; interpolation multi-type amplicon data storage 404 for storing created interpolation multi-type amplicon data; new sample electrophoretic analysis unit 108 for analyzing new samples 107 through electrophoresis; and new sample result data analysis unit 110 for searching uni-type amplicon data storage 402 and/or interpolation multi-type amplicon data storage 404 based on new sample electrophoresis result data 109 delivered by new sample electrophoretic analysis unit 108 to deliver a search result as new sample analysis result 111.

Here, uni-type amplicon sample preservation unit 101 preserves a plurality of uni-type amplicon samples, each of which is a DNA sample that includes one type of amplicon, and also holds STR counts in these samples for every amplicon samples. Selected samples 102 include one type of samples selected from uni-type amplicon sample preservation unit 101. The result of analyzing selected samples 102 by electrophoretic analysis unit 104 through electrophoresis is uni-type amplicon electrophoresis result data 401, and uni-type amplicon data storage 402 stores uni-type amplicon electrophoresis result data 401 and STR counts of amplicons corresponding to that uni-type amplicon electrophoresis result data 401 in a paired manner. In the fourth embodiment, all samples are selected from uni-type amplicon sample preservation unit 101, and designated as selected samples 102, respectively. The selected samples 102 are measured to determine how the result of electrophoresis varies thereby producing statistical data.

The fourth embodiment uses an interpolation method to find the electrophoresis result which would be indicated by samples including a plurality of amplicons. Accordingly, interpolation multi-type amplicon data creation unit 403 creates such data, i.e., interpolation multi-type amplicon data using a simulation method or the like from data stored in uni-type amplicon data storage 402, and preserves the created interpolation multi-type amplicon data in interpolation multi-type amplicon data storage 404.

New sample result data analysis unit 110 compares and analyzes new sample electrophoresis result data 109 which is the result of analyzing new samples 107 through electrophoresis, and data stored in interpolation multi-type amplicon data storage 404 to estimate STR counts of new samples 107, which is delivered as new sample analysis result 111.

In another example of the fourth embodiment, all uni-type amplicon samples stored in uni-type amplicon sample preservation unit 101 may not be used as selected samples 102, respectively, but some of samples may be selected from uni-type amplicon sample preservation unit 101 for use as selected samples 102.

Alternatively, in the fourth embodiment, new sample result data analysis unit 110, when it analyzes new sample electrophoresis result data 109, may use uni-type amplicon electrophoresis result data 401 stored in uni-type amplicon data storage 402 in addition to data stored in interpolation multi-type amplicon data storage 404.

«Fifth Embodiment»

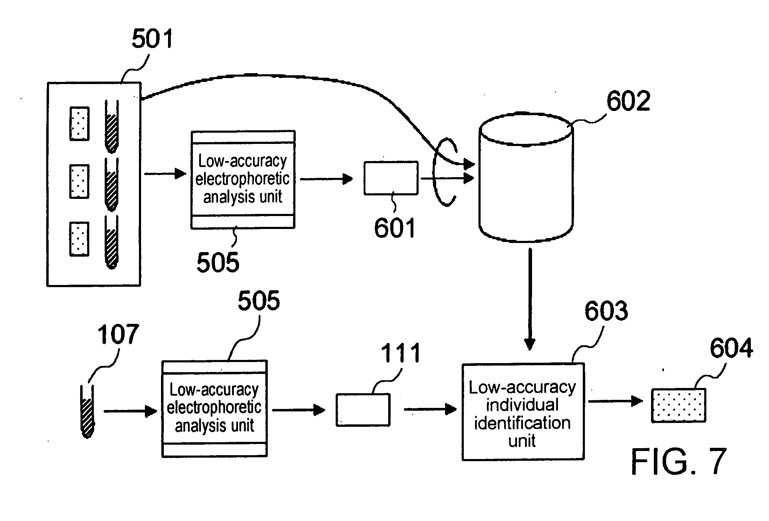

FIG. 7 shows the configuration of an individual identification apparatus according to a fifth embodiment of the present invention. This individual identification apparatus is similar to that of the first embodiment, but largely differs from that of the first embodiment in that low-accuracy electrophoretic analysis unit 505 is used for analyzing identifier-attached sample 501 instead of a high-accuracy electrophoretic analyzer. Low-accuracy electrophoretic analysis unit 505 analyzes each sample of identifier-attached samples 501 with a low accuracy, and delivers the result as low-accuracy identifier-attached sample analysis result 601. Low-accuracy identifier-attached sample analysis result 601 is stored in low-accuracy identifier-attached sample analysis data storage 602 together with identifiers for each individual of identifier-attached samples 501.

New samples 107 which are subjected to individual identification are analyzed by low-accuracy electrophoretic analysis unit 505 in a manner similar to the first embodiment, and as a result, new sample analysis result 111 is obtained. Low-accuracy individual identification unit 603 searches for entries having STR counts common to new sample analysis result 111 with reference to low-accuracy identifier-attached sample analysis data storage 602, and delivers found entries as low-accuracy individual identification result 604.

While the discrimination power correspondingly decreases as the analysis accuracy is lower in electrophoresis, it is possible to evaluate how much the discrimination power decreases, as described in the first embodiment. A reduction in resolution of electrophoretic analysis for identifier-attached samples 501 can be effectively treated as an analysis accuracy which does not decrease for identifier-attached samples 501 but further decreases for new sample analysis result 111. When the discrimination power is calculated on the assumption that the analysis accuracy further decreases in new sample analysis result 111 in this way, identifier-attached samples 501 may be analyzed by low-accuracy electrophoretic analysis unit 505 without causing any problem, provided that the calculated discrimination power is acceptable.

«Sixth Embodiment»

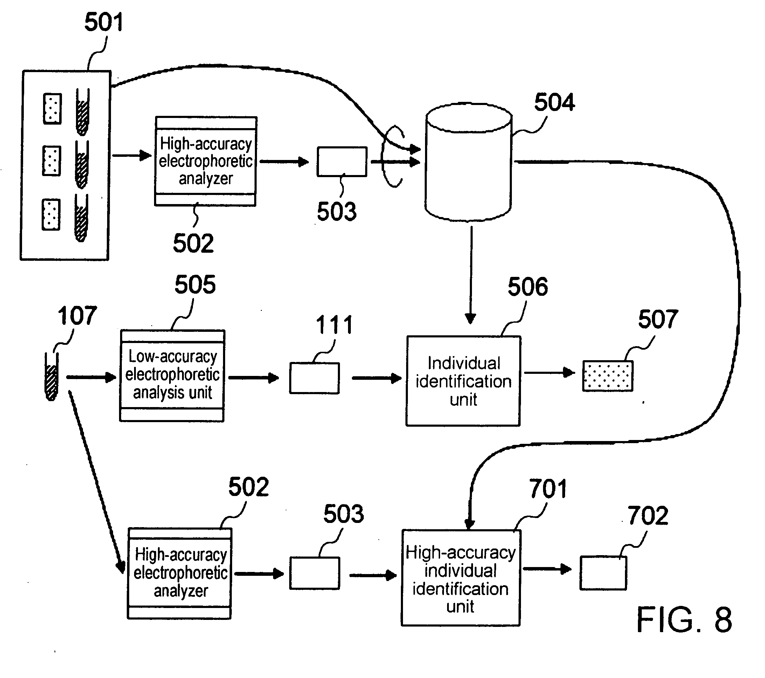

FIG. 8 shows the configuration of an individual identification apparatus according to a sixth embodiment of the present invention. This individual identification apparatus is similar to that of the first embodiment, but differs from the first embodiment in that when new samples 107 are analyzed by low-accuracy electrophoretic analysis unit 505, and are compared with data within identifier-attached sample analysis data storage 504 to obtain individual identification result 507, and new sample 107 is again analyzed by high-accuracy electrophoretic analyzer 502 when new samples 107 can match with STR counts of a plurality of individuals according to resulting individual identification result 507. New samples 107 are analyzed by high-accuracy electrophoretic analyzer 502 to produce sample result 503. This individual identification apparatus is provided with high-accuracy individual identification unit 701, and high-accuracy individual identification unit 701 searches for entries having STR counts common to sample analysis result 503 within entries in identifier-attached sample analysis data storage 504, based on sample analysis result 503 derived from new samples 107, and delivers the search result as high-accuracy individual identification result 702.

«Seventh Embodiment»

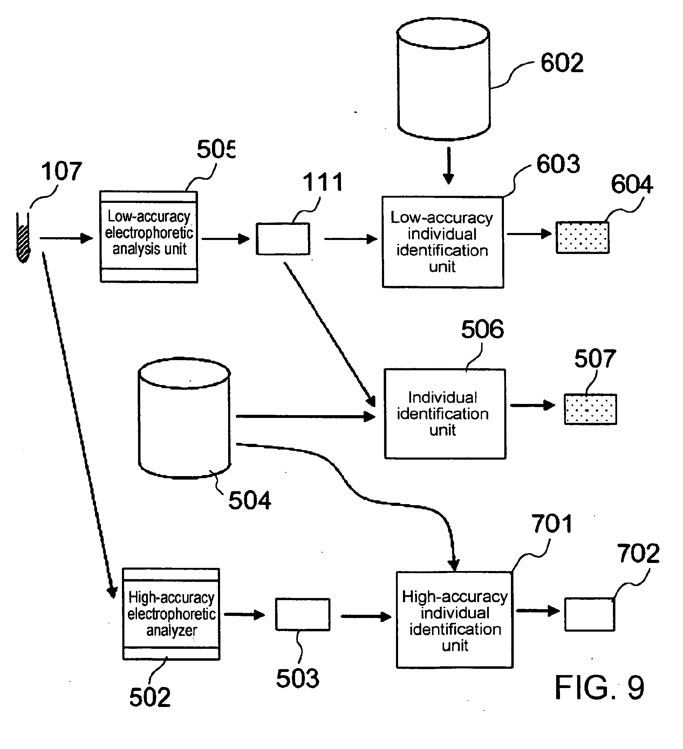

FIG. 9 shows the configuration of an individual identification apparatus according to a seventh embodiment of the present invention. This individual identification apparatus comprises: low-accuracy identifier-attached sample analysis data storage 602 in which low-accuracy identifier-attached sample analysis result 601 is stored in a procedure similar to the case of the fifth embodiment (see FIG. 7); and identifier-attached sample analysis data storage 504 in which sample analysis result 503 of identifier-attached samples 501 is stored in a procedure similar to the case of the sixth embodiment (see FIG. 8). Then, in this individual identification apparatus, in a manner similar to the case of the fifth embodiment, new samples 107 are first analyzed by low-accuracy electrophoretic analysis unit 505 to obtain new sample analysis result 111, and low-accuracy individual identification unit 603 searches low-accuracy identifier-attached sample analysis data storage 602 based on new sample analysis result 111 to deliver low-accuracy individual identification result 604. When there is one or a plurality of individually identified entries, a search is then made for an identifier which has a set of STR counts of new sample analysis result 111 that overlaps with a set of STR counts of each entry in identifier-attached sample analysis data storage 504 with reference to identifier-attached sample analysis data storage 504 by individual identification unit 506 based on previously produced new sample analysis result 111, and the search result is used as individual identification result 507, in a manner similar to the case of the first embodiment and the like.

When one or a plurality of entries has been searched and exists in individual identification result 507, a completely matching entry can exist in identifier-attached sample analysis data storage 504. For investigating this, next, new samples 107 are analyzed by high-accuracy electrophoretic analyzer 502 in a manner similar to the case of the sixth embodiment to obtain sample analysis result 503. High-accuracy individual identification unit 701 searches for entries which have STR counts common to sample analysis result 503 within entries in identifier-attached sample analysis data storage 504 based on sample analysis result 503 derived from new samples 107, and delivers the search result as high-accuracy individual identification result 702.

«Eighth Embodiment»

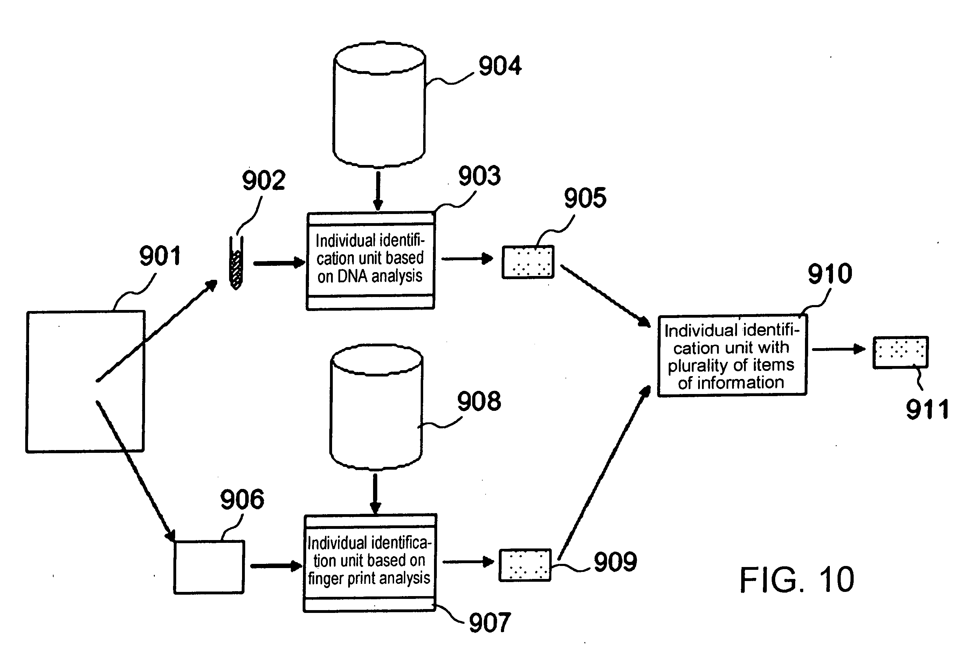

FIG. 10 shows the configuration of an individual identification apparatus according to the sixth embodiment of the present invention. This individual identification apparatus performs a DNA analysis and also performs an individual identification using other individual identification information (biometrics information) such as fingerprint and the like. Here, a description will be given of the case where an individual identification is made on new sample acquisition object 901, where new sample acquisition object 901 refers to an object from which DNA sample 902 and fingerprint sample 906 or the like can be sampled.

This individual identification apparatus comprises: individual identification unit 903 based on DNA analysis; identifier-attached DNA analysis data storage 904; individual identification unit 907 based on finger print analysis; identifier-attached finger print analysis data storage 908; and individual identification unit 910 using a plurality of items of information. Here, individual identification unit 903 based on DNA analysis is similar to the individual identification apparatus in any one of the embodiments described above, and analyzes DNA samples 902 (new samples 107 in each of the aforementioned embodiments), searches identifier-attached DNA analysis data storage 904 based on the analysis result, and delivers the search result as individual identification result 905 based on DNA analysis. Identifier-attached DNA analysis data storage 904 is comparable to identifier-attached sample analysis data storage 504 (or low-accuracy identifier-attached sample analysis data storage 602) in the aforementioned embodiments, and stores analysis results in DNA samples to which identifiers are attached, i.e., DNA samples the source of which is definite.

Likewise, identifier-attached finger print analysis data storage 908 stores the result of analyzing finger print data to which identifiers are attached, i.e., finger print data the source of which is definite. Individual identification unit 907 based on finger print analysis performs a finger print analysis on finger print samples 906 sampled from new sample acquisition object 901, and delivers information indicative of which individuals finger print samples 906 are identified, with reference to identifier-attached finger print analysis data storage 908, as finger print analysis based individual identification result 909. In this regard, since finger print analysis techniques are well known to those skilled in the art and are not directly related to the present invention, a detailed description thereon in omitted.

In this way, once individual identification result 905 base on DNA analysis and individual identification result 909 based on finger print analysis are derived, individual identification unit 910 using a plurality of items of information combines these individual identification results 905, 906 to deliver individual identification result 911 with a plurality of items of information. Since the individual identification apparatus of the eighth embodiment performs the individual identification by combining the result from the DNA analysis and the result from the finger print analysis or the like, the individual identification capabilities can be improved.

The eighth embodiment can employ, as other individual identification information combined with the DNA analysis result, information derived by individual identification techniques which utilizes an iris, a palm print, or a face and the like, other than the aforementioned finger print analysis information. Also, a plurality of combinations of these techniques are also possible. Since each of these analysis techniques is well known to those skilled in the art and is not directly related to the present invention, a detailed description thereon is omitted.

**Claims**

**1.** An individual identification method for identifying an individual by analyzing a DNA sample through electrophoresis, comprising:

a first analysis step of analyzing an identifier-attached DNA sample which is given an identifier for an individual; a step of storing a result obtained by analyzing the identifier-attached DNA sample together with a corresponding identifier in an identifier-attached sample analysis data storage; a second analysis step of analyzing a new sample which is a DNA sample subjected to individual identification with an accuracy lower than the accuracy when the identifier-attached DNA sample is analyzed, and using the result as a new sample analysis result; and a step of searching said identifier-attached sample analysis data storage based on the new sample analysis result.

2. The method according to claim 1, wherein, when the identifier-attached DNA sample and the new sample are analyzed, information related to base lengths of the samples are found through electrophoresis.

3. The method according to claim 2, wherein, upon analyzing the identifier-attached DNA sample and the new sample, information related to the number of times of repetition of micro-satellites in the sample is captured.

4. The method according to claim 2 or 3, wherein the analysis accuracy in said first analysis step is an accuracy with which two DNAs can be identified, where the two DNAs differ in base length by a conceivably minimal amount of change of a base length in the new sample, and the analysis accuracy in said second analysis step is an accuracy with which the two DNA cannot be identified, where the two DNAs differ in base length by the minimum amount of change.

5. The method according to any one of claims 1 to 4, wherein said second analysis step comprises:

a step of selecting a plurality of samples in an arbitrary combination from a group of samples each including one type of amplicon, and mixing selected samples to generate a multi-type amplicon sample; a third analysis step of analyzing the multi-type amplicon sample through electrophoresis; a step of storing a result obtained in said third analysis step and base length information of the multi-type amplicon sample in a multi-type amplicon data storage in a paired manner; a fourth analysis step of analyzing the new sample through electrophoresis to obtain new sample electrophoresis result data; and a search step of searching said multi-type amplicon data storage based on the new sample electrophoresis result data, and using the result as the new sample analysis result.

6. The method according to claim 5, further comprising the step of generating base length information through interpolation with respect to a combination, the analysis result of which has not been stored in said multi-type amplicon data storage, within combinations of amplicons, and storing the base length information in an interpolation data storage, wherein said multi-type amplicon data storage and said interpolation data storage are searched on the basis of the new sample electrophoresis result data in said search step, and the result is used as the new sample analysis result.

7. The method according to any one of claims 1 to 4, wherein said second analysis step comprises:

a step of selecting samples based on part of combinations within combinations available from a group of samples each including one type of amplicon, and mixing the selected samples to generate a multi-type amplicon sample; a third analysis step of analyzing the multi-type amplicon sample through electrophoresis; a step of storing a result obtained in said third analysis step and base length information of the multi-type amplicon sample in a multi-type amplicon data storage in a paired manner; a fourth analysis step of analyzing the new sample through electrophoresis to obtain new sample electrophoresis result data; and a step of parameterizing the new sample electrophoresis result data with respect to a change in base length in the new sample, with reference to said multi-type amplicon data storage, searching said multi-type amplicon data storage based on the result of the parameterization and the new sample electrophoresis result data, and using the result of the search as the new sample analysis result.

8. The method according to any one of claims 1 to 4, further comprising the step of analyzing the new sample with a similar accuracy to that used in said first analysis step when a pertinent entry is found from said identifier-attached sample analysis data storage as a result of searching said identifier-attached sample analysis data storage based on the new sample analysis result, and searching said identifier-attached sample analysis data storage using the result by the analysis to obtain an individual identification result.

9. An individual identification method for identifying an individual by analyzing a DNA sample through electrophoresis, comprising:

a first analysis step of analyzing an identifier-attached DNA sample which is given an identifier for an individual to obtain information on a base length of the identifier-attached DNA sample;
a step of storing a result obtained by analyzing the identifier-attached DNA sample together with a corresponding identifier in an identifier-attached sample analysis data storage;
a second analysis step of analyzing a new sample which is a DNA sample subjected to individual identification, and using a result including information related to a base length of the new sample as a new sample analysis result; and
a step of searching said identifier-attached sample analysis data storage based on the new sample analysis result,

wherein accuracies in said first analysis step and said second analysis step are accuracies with which two DNAs cannot be identified if the two DNAs differ in base length by a conceivable minimal amount of change of a base length in a DNA sample subjected to individual identification.

10. The method according to claim 9, wherein, upon analyzing the identifier-attached DNA sample and the new sample, information related to the number of times of repetition of micro-satellites in the sample is captured.

11. A plural information based individual identification method comprising:

a first individual identification step of implementing the method according to any one of claims 1 to 10 using a DNA derived from a new sample acquisition object as a DNA sample subjected to the individual identification;
a second individual identification step of identifying an individual using biometrics information other than DNA derived from the new sample acquisition object; and
a third individual identification step of performing individual identification based on a result obtained in said first individual identification step and a result obtained in said second individual identification step.

12. The plural information based individual identification method according to claim 11, wherein said biometrics information is finger print information.

13. An individual identification apparatus for identifying an individual by analyzing a DNA sample through electrophoresis, comprising:

first analysis means for analyzing an identifier-attached DNA sample which is given an identifier for an individual;
an identifier-attached sample analysis data storage for storing a result obtained by analyzing the identifier-attached DNA sample by said first analysis means together with a corresponding identifier;
second analysis means having an analysis accuracy lower than said first analysis means, for analyzing a new sample which is a DNA sample subjected to individual identification, and using the result as a new sample analysis result; and
identification means for searching said identifier-attached sample analysis data storage based on the new sample analysis result to obtain an individual identification result.

14. The apparatus according to claim 13, wherein both said first and second analysis means find information related to base lengths of the samples through electrophoresis.

15. The apparatus according to claim 14, wherein, when said first and second analysis means analyze the identifier-attached DNA sample and the new sample, respectively, said first and second analysis means capture information related to the number of times of repetition of micro-satellites in the sample.

16. The apparatus according to claim 14 or 15, wherein the analysis accuracy of said first analysis means is an accuracy with which two DNAs can be identified, where the two DNAs differ in base length by a conceivably minimal amount of change of a base length in the new sample, and the analysis accuracy of said second analysis means is an accuracy with which the two DNA cannot be identified, where the two DNAs differ in base length by the minimum amount of change.

17. The apparatus according to any one of claims 13 to 16, wherein said second analysis means comprises:

a uni-type amplicon sample preservation unit for containing a group of samples each including one type of amplicon;

a first electrophoretic analysis unit for selecting a plurality of samples in an arbitrary combination from said uni-type amplicon sample preservation unit, and analyzing a multi-type amplicon sample produced by mixing selected samples through electrophoresis;

a multi-type amplicon data storage for storing a result obtained in said first electrophoretic analysis unit and base length information in the multi-type amplicon sample;

a second electrophoretic analysis unit for analyzing the new sample through electrophoresis to obtain new sample electrophoresis result data; and

a data analysis unit for searching said multi-type amplicon data storage based on the new sample electrophoresis result data, and using the result as the new sample analysis result.

18. The apparatus according to claim 17, further comprising:

an interpolation data creation unit for generating base length information through interpolation with respect to a combination, the analysis result of which has not been stored in said multi-type amplicon data storage, within combinations of amplicons; and

an interpolation data storage for storing the base length information created by said interpolation data creation unit,

wherein said data analysis unit searches said multi-type amplicon data storage and said interpolation data storage based on the new sample electrophoresis result data, and uses the result as the new sample analysis result.

19. The apparatus according to any one of claims 13 to 16, wherein said second analysis means comprises:

a first electrophoretic analysis unit for analyzing through electrophoresis a multi-type amplicon sample generated by selecting samples based on part of combinations within combinations available from a group of samples each including one type of amplicon, and mixing the selected samples;

a multi-type amplicon data storage for storing a result obtained by said first electrophoretic analysis unit and base length information of the multi-type amplicon sample in a paired manner;

a second electrophoretic analysis unit for analyzing the new sample through electrophoresis to obtain new sample electrophoresis result data; and

a data analysis unit for parameterizing the new sample electrophoresis result data with respect to a change in base length in the new sample, with reference to said multi-type amplicon data storage, searching said multi-type amplicon data storage based on the result of the parameterization and the new sample electrophoresis result data, and using the result of the search as the new sample analysis result.

20. The apparatus according to any one of claims 13 to 16, further comprising:

third analysis means having an accuracy similar to said first analysis means; and

high-accuracy identification means for searching said identifier-attached sample analysis data storage using a result by said third analysis means to obtain an individual identification result,

wherein said third analysis means analyzes the new sample when a pertinent entry is found from said identifier-attached sample analysis data storage as a result of said identification means searching said identifier-attached sample analysis data storage based on the new sample analysis result.

21. An individual identification apparatus for identifying an individual by analyzing a DNA sample through electrophoresis, comprising:

first analysis means for analyzing an identifier-attached DNA sample which is given an identifier for an individual to obtain information on a base length of the identifier-attached DNA sample;

an identifier-attached sample analysis data storage for storing a result obtained by analyzing the identifier-attached DNA sample together with a corresponding identifier;
second analysis means for analyzing a new sample which is a DNA sample subjected to individual identification, and using a result including information related to a base length of the new sample as a new sample analysis result; and
identification means for searching said identifier-attached sample analysis data storage based on the new sample analysis result,

wherein accuracies of analysis in said first analysis means and said second analysis means are accuracies with which two DNAs cannot be identified if the two DNAs differ in base length by a conceivable minimal amount of change of a base length in a DNA sample subjected to individual identification.

22. The apparatus according to claim 21, wherein, upon analyzing the identifier-attached DNA sample and the new sample, said first and second analysis means obtain information related to the number of times of repetition of micro-satellites in the samples.

23. A plural information based individual identification apparatus comprising:

first individual identification means including the apparatus according to any one of claims 13 to 22, for using a DNA derived from a new sample acquisition object as a DNA sample subjected to the individual identification;
second individual identification means for identifying an individual using biometrics information other than DNA derived from the new sample acquisition object; and
third individual identification means for performing individual identification based on a result obtained in said first individual identification means and a result obtained in said second individual identification means.

24. The plural information based individual identification apparatus according to claim 23, wherein said biometrics information is finger print information.

FIG. 1

FIG. 2

EP 2 012 116 A1

FIG. 3

FIG. 4

FIG. 5

EP 2 012 116 A1

**101**

**102**

**104**

Electro-
phoretic
analysis
unit

**401**

**402**

**403**

**404**

**107**

**108**

New sample
electropho-
retic analysis
unit

**109**

**110**

New sample
result data
analysis unit

**111**

FIG. 6

FIG. 7

FIG. 8

FIG. 9

FIG. 10

EP 2 012 116 A1

## INTERNATIONAL SEARCH REPORT

| | International application No. |
|---|---|
| | PCT/JP2007/058055 |

A.  CLASSIFICATION OF SUBJECT MATTER
*G01N27/447*(2006.01)i, *C12M1/00*(2006.01)i, *C12Q1/68*(2006.01)i

According to International Patent Classification (IPC) or to both national classification and IPC

B.  FIELDS SEARCHED

Minimum documentation searched (classification system followed by classification symbols)
G01N27/447, C12M1/00, C12Q1/68

Documentation searched other than minimum documentation to the extent that such documents are included in the fields searched
Jitsuyo Shinan Koho        1922-1996   Jitsuyo Shinan Toroku Koho   1996-2007
Kokai Jitsuyo Shinan Koho  1971-2007   Toroku Jitsuyo Shinan Koho   1994-2007

Electronic data base consulted during the international search (name of data base and, where practicable, search terms used)
JST7580(JDream2), JSTPlus(JDream2)

C.  DOCUMENTS CONSIDERED TO BE RELEVANT

| Category* | Citation of document, with indication, where appropriate, of the relevant passages | Relevant to claim No. |
|---|---|---|
| Y<br><br>A | Kentaro KASAI, "Hanzai Sosa ni Okeru DNA-gata Kantei", Gene & medicine, Kabushiki Kaisha Medical Do Hakko, 10 November, 1999 (10.11.99) Hakko, Vol.3, No.4, pages 726 to 730 | 1-4,8-16, 20-24<br>5-7,17-19 |
| Y<br><br>A | Yukio ITAKURA, "DNA Ninsho Hoshiki to Sono Kadai", Chemical Engineering, Kabushiki Kaisha Kagaku Kogyosha Hakko, 01 July, 2005 (01.07.05) Hakko, Vol.50, No.7, pages 500 to 507 | 1-4,8-16, 20-24<br>5-7,17-19 |
| Y | JP 2003-150720 A  (Dainippon Printing Co., Ltd.), 23 May, 2003 (23.05.03), Claims (Family: none) | 11-12,23-24 |

| ☒ | Further documents are listed in the continuation of Box C. | ☐ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 04 July, 2007 (04.07.07) | 17 July, 2007 (17.07.07) |

| Name and mailing address of the ISA/ | Authorized officer |
|---|---|
| Japanese Patent Office | |
| Facsimile No. | Telephone No. |

Form PCT/ISA/210 (second sheet) (April 2005)

## INTERNATIONAL SEARCH REPORT

International application No.

PCT/JP2007/058055

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2001-338135 A (Hidekazu KIHARA), 07 December, 2001 (07.12.01), Claims (Family: none) | 11-12,23-24 |
| Y | JP 2001-168855 A (Sony Corp.), 22 June, 2001 (22.06.01), Claims (Family: none) | 11-12,23-24 |
| A | JP 2003-245098 A (Hitachi, Ltd.), 02 September, 2003 (02.09.03), Full text & US 2003/0175759 A1 | 1-24 |

Form PCT/ISA/210 (continuation of second sheet) (April 2005)

**REFERENCES CITED IN THE DESCRIPTION**

**Patent documents cited in the description**

- JP 2002253203 A
- JP 2003245098 A
- JP 2004073188 A
- JP 2005013226 A
- JP 2005160302 A
- JP 2005237334 A
- JP 2005307216 A
- JP 11118760 A
- WO 9715690 A
- WO 9835060 A
- WO 0114590 A
- WO 0208469 A
- WO 0266650 A
- WO 0306692 A
- WO 0286794 A
- JP 2000500647 A
- JP 2001511018 A
- JP 2003507049 A
- JP 2004516455 A
- JP 2004531235 A
- JP 2004535198 A
- JP 2005509844 A

**Non-patent literature cited in the description**

- **BRUCE BUDOWLE.** Genotype Profiles for Six Population Groups at the 13 CODIS Short Tandem Repeat Core Loci and Other PCRB Based Loci. *Forensic Science,* July 1999, vol. 1 (2
- Forensic DNA Typing, Second Edition: Biology, Technology, and Genetics of STR Markers. 2005, 85-117
- **JOHN M. BUTLER.** Forensic DNA Typing, Second Edition: Biology, Technology, and Genetics of STR Markers. 2005, 85-117345-370373-386